(19) Europäisches Patentamt
European Patent Office
Office européen des brevets

(11) EP 1 444 974 A2

(12) DEMANDE DE BREVET EUROPEEN

(43) Date de publication:
**11.08.2004 Bulletin 2004/33**

(51) Int Cl.7: **A61K 7/11**, A61K 7/06

(21) Numéro de dépôt: **03292869.9**

(22) Date de dépôt: **19.11.2003**

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **13.12.2002 FR 0215855**
**21.03.2003 FR 0303506**

(71) Demandeur: **L'OREAL**
**75008 Paris (FR)**

(72) Inventeur: **Mougin, Nathalie**
**75011 Paris (FR)**

(74) Mandataire: **Dodin, Catherine**
**L'OREAL - D.I.P.I.**
**25-29 Quai Aulagnier**
**92600 Asnières (FR)**

(54) **Composition capillaire comprenant un copolymère filmogène à gradient, composition aérosol la comprenant et procédés de traitement**

(57) La présente demande concerne une composition cosmétique capillaire comprenant, dans un milieu cosmétiquement acceptable, au moins un copolymère filmogène à gradient comprenant au moins deux monomères différents, et présentant un indice de polydispersité en masse (Ip) inférieur ou égal à 2,5, ladite composition étant apte à former un film présentant une déformation à la rupture $\varepsilon_r$ comprise entre 5% et 2500%, et/ou un module d'Young compris entre 0,5 et 1200 MPa et/ou une recouvrance élastique instantanée $\varepsilon_i$ supérieure ou égale à 10%.

L'invention concerne également une composition aérosol conditionnée dans un dispositif aérosol, comprenant un agent propulseur et une composition capillaire telle que définie ci-dessus, ainsi qu'un procédé de traitement des cheveux, notamment un procédé de coiffage, consistant à appliquer sur ceux-ci la composition capillaire ou la composition aérosol ci-dessus.

EP 1 444 974 A2

## Description

La présente invention a trait à de nouvelles compositions cosmétiques capillaires comprenant des copolymères particuliers, notamment des copolymères amphiphiles à gradient, de préférence solubles ou dispersibles dans l'eau et/ou dans les solvants organiques.

Les compositions de coiffagè conditionnées sous forme de spray aérosol contiennent généralement une fraction importante d'alcool. Or, les produits cosmétiques à haute teneur en alcool font l'objet d'une surveillance particulière, notamment aux Etats Unis, suite à la sensibilisation récente de l'opinion publique aux problèmes écologiques résultant de l'émission de produits organiques volatils dans l'atmosphère.

Une solution permettant de diminuer la quantité d'alcool, voire de supprimer totalement l'alcool des formules, est le remplacement par une quantité équivalente d'eau. Cette introduction de quantités importantes d'eau dans les sprays aérosols destinés à la fixation des cheveux, tels que des laques, conduit toutefois à une déformation importante indésirable de la chevelure et à une dégradation des propriétés cosmétiques. Par ailleurs, la plupart des agents propulseurs de type hydrocarbures sont incompatibles avec l'eau et leur utilisation dans des compositions à forte teneur en eau est de ce fait généralement impossible.

Les polymères radicalaires conventionnels c'est à dire ceux obtenus par polymérisation radicalaire classique peuvent être utilisés dans cet axe, en solution dans 'l'éthanol, dans l'eau ou dans un mélange eau/éthanol.

Malgré tout, de nombreux polymères utilisés actuellement ne sont pas ou difficilement compatibles avec l'eau et précipitent. En effet, même s'ils comportent des motifs hydrophiles, ils présentent par ailleurs l'inconvénient d'avoir une polydispersité en composition importante ce qui implique que certaines chaînes polymériques comportent les motifs hydrophiles nécessaires à la solubilité alors que d'autres ne comportent pas ces motifs et donc sont insolubles dans les milieux hydrophiles. Il en résulte des problèmes de démixtion au sein de la composition.

La présente invention a pour but de pallier les inconvénients de l'art antérieur en proposant une composition cosmétique capillaire comprenant des polymères particuliers, qui évitent les problèmes de demixtion au sein de la formule tout en permettant d'apporter les propriétés cosmétiques recherchées.

Un objet de la présente invention est une composition cosmétique capillaire comprenant, dans un milieu cosmétiquement acceptable, au moins un copolymère filmogène à gradient comprenant au moins deux monomères différents, et présentant un indice de polydispersité en masse (Ip) inférieur ou égal à 2,5 de préférence compris entre 1,1 et 2,3, notamment entre 1,15 et 2,0, voire entre 1,2 et 1,9

ou 1,8, ladite composition étant apte à former un film présentant au moins une des caractéristiques suivantes:

- une déformation à la rupture $\varepsilon_r$ comprise entre 5% et 2500%, de préférence entre 10% et 2000% et encore mieux entre 15% et 1000%, et/ou
- un module d'Young compris entre 0,5 et 1200 MPa, de préférence entre 1 et 1000 MPa, préférentiellement entre 2 et 800 MPa, et/ou
- une recouvrance élastique instantanée $\varepsilon_i$ supérieure ou égale à 10%, de préférence supérieure ou égale à 25%, et mieux supérieure ou égale à 35%, notamment comprise entre 10-100%, par exemple 25-98%, voire 35-95%.

Un autre objet de l'invention est une composition aérosol conditionnée dans un dispositif aérosol et comprenant un agent propulseur et une composition capillaire telle que définie ci-après.

Un autre objet de l'invention est un procédé de traitement des cheveux, notamment un procédé de coiffage, consistant à appliquer sur ceux-ci la composition capillaire ci-dessus et/ou à vaporiser sur ceux-ci la composition aérosol ci-dessus, et éventuellement à laisser sécher la chevelure ainsi traitée.

Comme les copolymères à gradient selon l'invention présentent une faible dispersité en composition, toutes les chaînes présentant quasiment les mêmes structures, elles sont donc compatibles entre elles; il en résulte que les compositions cosmétiques comprenant ces copolymères ne présentent pas les inconvénients et limitations des compositions de l'art antérieur.

Notamment les copolymères selon l'invention présentent l'avantage d'être facilement manipulables dans l'eau ou dans un milieu solvant organique, tout en conservant des propriétés rhéologiques intéressantes.

Par ailleurs, les copolymères à gradients comprennent une quantité de monomère hydrophile adéquate, ce qui permet d'obtenir une solubilité plus aisée : la solubilité est favorisée car toutes les chaînes ont la même composition.

Les copolymères selon l'invention sont des copolymères à gradient, qui comprennent au moins deux monomères différents, et qui présentent une polydispersité en masse faible et de préférence une polydispersité en composition faible.

La polydispersité en masse peut être illustrée à l'aide de l'indice de polydispersité en masse (Ip) du copolymère, qui est égal au rapport de la masse moléculaire moyenne en poids (Mw) sur la masse moléculaire moyenne en nombre (Mn).

Une faible dispersité en masse traduit des longueurs de chaînes approximativement identiques, ce qui est le cas pour

les copolymères selon la présente invention.
Le copolymère à gradient selon l'invention possède un indice de polydispersité en masse inférieur ou égal à 2,5 de préférence compris entre 1,1 et 2,3, notamment entre 1,15 et 2,0, voire entre 1,2 et 1,9 ou 1,8.

Par ailleurs, la masse moléculaire moyenne en poids du copolymère à gradient est de préférence comprise entre 5000 g/mol et 1 000 000 g/mol, notamment entre 5500 g/mol et 800 000 g/mol, et encore mieux entre 6000 g/mol et 500 000 g/mol.
De préférence, la masse moléculaire moyenne en nombre du copolymère à gradient est comprise entre 5000 g/mol et 1 000 000 g/mol, notamment entre 5500 g/mol et 800 000 g/mol, et encore mieux entre 6000 g/mol et 500 000 g/mol.
On détermine les masses moléculaires moyennes en poids (Mw) et en nombre (Mn) par chromatographie liquide par perméation de gel (GPC), éluant THF, courbe d'étalonnage établie avec des étalons de polystyrène linéaire, détecteur réfractométrique).

Le copolymère à gradient selon l'invention présente également préférentiellement une faible dispersité en composition. Ceci signifie que toutes les chaînes de copolymères ont une composition (c'est-à-dire un enchaînement de monomères) approximativement analogue et sont donc homogènes en composition.
Afin de montrer que toutes les chaînes de copolymères ont une composition analogue, on utilisera avantageusement la chromatographie d'adsorption liquide (ou LAC) qui permet de séparer les chaînes de copolymères, non pas selon leur poids moléculaire mais selon leur polarité. Cette dernière reflète la composition chimique des polymères constituant le matériau, les monomères étant connus.
On pourra se reporter à la publication Macromolecules (2001), 34, 2667 qui décrit la technique LAC.
On peut notamment définir la polydispersité en composition à partir de la courbe de chromatographie d'adsorption (LAC) (courbe représentant la proportion de polymères en fonction du volume d'élution): si l'on nomme "$V^{1/2}$ min" la valeur minimum du volume d'élution à mi-hauteur de la courbe, et "$V^{1/2}$ max" la valeur maximum du volume d'élution à mi-hauteur de la courbe, la polydispersité en composition est considérée comme faible si la différence ($V^{1/2}$ max - $V^{1/2}$ min) est inférieure ou égale à 3,5, notamment comprise entre 1 et 2,8 et mieux entre 1,2 et 2,5.
Par ailleurs, la courbe LAC peut être définie par une courbe de Gauss de formule:

$$y = \frac{A}{w\sqrt{\frac{\pi}{2}}} \times e^{-2\frac{(x-x_0)^2}{w^2}} + y_o$$

dans laquelle :

- $x_0$ est la valeur de x (volume d'élution) au centre du pic
- w est égal à 2 fois la déviation standard de la distribution gaussienne (soit $2\sigma$) ou encore correspond à approximativement à 0,849 fois la largeur du pic à mi-hauteur
- A représente la surface sous le pic
- $y_o$ est la valeur de y correspondant à $x_0$.

La dispersité en composition peut également être définie par la valeur de w telle que définie ci dessus. De préférence, ladite valeur w est comprise entre 1 et 3, notamment entre 1,1 et 2,3 et encore mieux entre 1,1 et 2,0.

Les copolymères à gradient selon l'invention peuvent être obtenus par polymérisation vivante ou pseudo-vivante.
Pour mémoire, nous rappelons que la polymérisation vivante est une polymérisation pour laquelle la croissance des chaînes polymériques ne cesse qu'avec la disparition du monomère. La masse moyenne en nombre (Mn) croît avec la conversion. La polymérisation anionique est un exemple typique de polymérisation vivante. De telles polymérisations conduisent à des copolymères dont la dispersité en masse est faible c'est-à-dire à des polymères d'indice de polydispersité en masse (Ip) généralement inférieur à 2.
La polymérisation pseudo-vivante est, quant à elle, associée à la polymérisation radicalaire contrôlée. Parmi les principaux types de polymérisation radicalaire contrôlée, on peut citer :

- la polymérisation radicalaire contrôlée par des nitroxydes. On peut notamment se référer aux demandes de brevet WO96/24620 et WO00/71501 qui décrivent les outils de cette polymérisation et leur mise en oeuvre, ainsi qu'aux articles publiés par Fischer (Chemical Reviews, 2001, *101*, 3581), par Tordo et Gnanou (J. Am. Chem. Soc. 2000, 122, 5929) et Hawker (J. Am. Chem. Soc. 1999, *121*, 3904);
- la polymérisation par transfert d'atome radicalaire, notamment décrite dans la demande WO96/30421 et qui procède par l'insertion réversible sur un complexe organométallique dans une liaison de type carbone-halogène;

- la polymérisation radicalaire contrôlée par des dérivés soufrés de type xanthate, dithioesters, trithiocarbonates ou carbamates, telle que décrite dans les demandes FR2821620, WO98/01478, WO99/35177, WO98/58974, WO99/31144, WO97/01478 et dans la publication de Rizzardo & al. (Macromolecules, 1998, 31, 5559).

La polymérisation radicalaire contrôlée désigne des polymérisations pour lesquelles les réactions secondaires qui conduisent habituellement à la disparition des espèces propageantes (réaction de terminaison ou transfert) sont rendues très peu probables par rapport à la réaction de propagation grâce à un agent de contrôle des radicaux libres. L'imperfection de ce mode de polymérisation réside dans le fait que lorsque les concentrations en radicaux libres deviennent importantes par rapport à la concentration en monomère, les réactions secondaires redeviennent déterminantes et tendent à élargir la distribution des masses.

Grâce à ces modes de polymérisation, les chaînes polymériques des copolymères à gradient selon l'invention croissent simultanément et donc incorporent à chaque instant les mêmes ratio de co-monomères. Toutes les chaînes possèdent donc les mêmes structures ou des structures proches, d'où une faible dispersité en composition. Ces chaînes possèdent également un indice de polydispersité en masse faible.

Les copolymères à gradient sont des copolymères présentant une évolution du ratio des différents monomères tout au long de la chaîne. La distribution dans les chaînes polymériques des co-monomères dépend de l'évolution pendant la synthèse des concentrations relatives des co-monomères.

Les copolymères selon l'invention comprennent au moins deux monomères différents dont la concentration le long de la chaîne polymérique change graduellement et de façon systématique et prédictible.

Ceci signifie que toutes les chaînes polymériques ont au moins un monomère Mi pour lequel, quelque soit la position normalisée x sur la chaîne polymérique, il y a une probabilité non nulle de retrouver ce monomère Mi le long de la chaîne. Une des caractéristiques permettant de définir les copolymères à gradient est le fait qu'à tout instant de la polymérisation, toutes les chaînes sont soumises à la présence de l'ensemble de tous les monomères. Ainsi, dans le milieu réactionnel, la concentration de chaque monomère est toujours non nulle, à tout instant de la polymérisation.

Ceci permet de différencier les copolymères selon l'invention des polymères-blocs usuels dans lesquels l'évolution des monomères le long de la chaîne polymérique n'est pas systématique par exemple pour un dibloc AB, au sein de la séquence A, la concentration en l'autre monomère B est toujours nulle.

Dans le cas des polymères statistiques, l'évolution des monomères le long de la chaîne polymérique ne sera pas non plus graduelle, systématique et prédictible.

Comme illustré par le schéma ci-après, un polymère statistique obtenu par polymérisation radicalaire classique de deux monomères se distingue d'un copolymère à gradient, par la répartition des monomères qui n'est pas identique sur toutes les chaînes, et par la longueur desdites chaînes qui n'est pas identique pour toutes les chaînes.

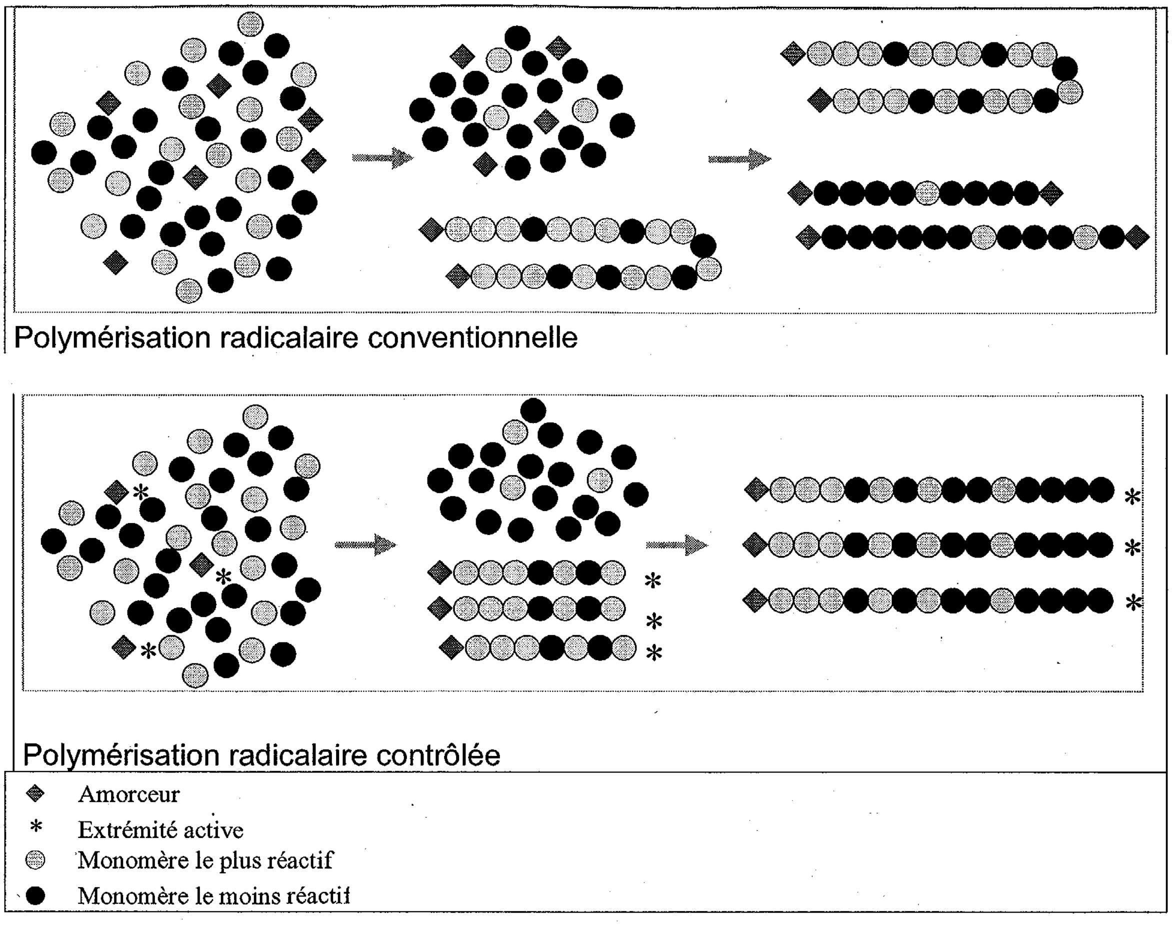


Pour une description théorique des copolymères à gradient, il est possible de se reporter aux publications suivantes :

T. Pakula et al., Macromol. Theory Simul. 5, 987-1006 (1996) ;
A. Aksimetiev et al. J. of Chem. Physics 111, n°5 ;
M. Janco, J. Polym. Sci., Part A: Polym. Chem. (2000), 38(15), 2767-2778 ;
M. Zaremski et al., Macromolecules (2000), 33(12), 4365-4372 ;
K. Matyjaszewski & al., J. Phys. Org. Chem. (2000), 13(12), 775-786 ;
Gray, Polym. Prepr. (Am. Chem. Soc., Div. Polym. Chem.) (2001), 42(2), 337-338 ;
K. Matyjaszewski, Chem. Rev. (Washington, D. C.) (2001), 101(9), 2921-2990.

Parmi les copolymères à gradient, on peut distinguer les copolymères à gradient naturel, et les copolymères à gradient artificiel.
Un copolymère à gradient naturel est un copolymère à gradient synthétisé en batch à partir d'un mélange initial des co-monomères. La distribution dans la chaîne des divers monomères suit une loi déduite de la réactivité relative et des concentrations initiales de monomères. Ces copolymères constituent la classe la plus simple de copolymères à gradient car c'est le mélange initial qui définit la propriété finale de produit.
Un copolymère à gradient artificiel est un copolymère dont on fait varier la concentration en monomères durant la synthèse par un artifice de procédé. Dans ce cas, on passe d'un mélange de monomères à un autre dans la chaîne du fait d'un changement subit et brusque des monomères dans le milieu réactionnel (stripping du premier mélange ou addition d'au moins un nouveau monomère). Il peut même y avoir une disparition nette d'un ou plusieurs des monomères au profit d'un ou plusieurs autres.
La caractérisation expérimentale du gradient est apportée par la mesure en cours de polymérisation de la composition chimique du polymère. Cette mesure est faite de façon indirecte en déterminant l'évolution de la teneur des différents monomères à tout instant. Elle peut être faite par RMN et UV, par exemple.

En effet, pour les polymères préparés par polymérisation vivante ou pseudo-vivante, la longueur des chaînes est linéairement liée à la conversion.
En prélevant un échantillon de la solution de polymérisation, à différents instants de la polymérisation et en mesurant la différence de teneur en chaque monomère, on accède ainsi à la composition du gradient.

Dans le polymère à gradient, la distribution des compositions des chaînes est étroite. En particulier il n'existe pas de recouvrement entre le pic du copolymère à gradient et ceux des homopolymères respectifs. Ceci signifie que le matériau obtenu en gradient est constitué de chaînes polymères de même composition alors qu'en polymérisation statistique classique, différentes sortes de chaîne coexistent, y compris celles des homopolymères respectifs.

Il est possible de caractériser les copolymères à gradient par un vecteur caractéristique de chaque copolymère.
En effet, sachant qu'il existe une infinité de polymères caractérisés par une composition chimique donnée, pour préciser un polymère il est possible de décrire la répartition des monomères le long de la chaîne. Ce qui implique une description à plusieurs variables. Ce vecteur est un point de l'espace des compositions chimiques.
Le terme exact est que G est un vecteur dont les coordonnées sont les concentrations des monomères le long de la chaîne polymérique. Ces concentrations sont définies par les règles des coefficients de réactivité de chacun des monomères, et donc sont liées à la concentration des monomères libres pendant la synthèse : du moment que le monomère n'est pas en concentration nulle dans le mélange réactionnel, il n'est pas en concentration nulle dans le polymère.
Il est donc possible de caractériser les copolymères à gradient par la fonction G(x) qui définit le gradient de composition :

$$\vec{G}(x) = \Sigma\overrightarrow{[Mi](x)}$$

dans laquelle :

- x désigne une position normalisée sur la chaîne polymère et
- [Mi](x) est la concentration relative, en cette position x, du monomère Mi exprimée en % en moles.

La fonction G(x) décrit donc localement la composition du copolymère à gradient.
Deux copolymères peuvent avoir une composition globale équivalente mais des répartitions locales des monomères très différentes, donc des gradients différents.
Par exemple, dans le cas d'un dibloc AB (50/50), la fonction [A] vaut 1 jusqu'à x=1/2 puis 0 ensuite.
Les facteurs déterminant le gradient sont d'une part les coefficients de réactivité relatifs de chaque monomère (appelé $r_i$ pour le monomère Mi) qui dépendent principalement du type de procédé de synthèse employé (homogène, dispersé) et des solvants, et d'autre part les concentrations initiales de chacun des monomères, ainsi que les ajouts éventuels de monomères au cours de la polymérisation.
Ainsi, si l'on considère, à titre d'exemple, un copolymère à gradient de styrène (M1) ayant un coefficient de réactivité relatif $r_1$= 0,418 et de l'acide méthacrylique (M2), avec $r_2$=0,6, dans un système de polymérisation homogène.
La variation des concentrations initiales en styrène et en acide méthacrylique permet l'obtention des différents copolymères à gradients ayant des chaînes de structures complètement différentes.
Lorsque la concentration initiale en acide méthacrylique est de 10% en poids, on obtient au final un copolymère à gradient très faible dont on ne peut attendre une nanostructuration. Lorsque cette concentration initiale est de 20% en poids, on obtient un copolymère à gradient ayant une "tête" hydrophile et une "queue" hydrophobe avec un gradient suffisamment prononcé pour conduire à une nanostructuration. Lorsque cette concentration est de 50% en poids, les monomères étant isoréactifs dans ces conditions, le copolymère obtenu est du type alterné.
Bien que les copolymères décrits soient tous des copolymères à gradient de styrène et d'acide méthacrylique, la différence de concentration initiale des monomères conduit à des chaînes de structures complètement différentes conférant aux copolymères des propriétés différentes. Cet exemple illustre donc l'importance des compositions initiales de monomère sur l'arrangement le long de la chaîne des différents monomères.
Dans le cas d'un copolymère à gradient styrène/acide méthacrylique, les différents polymères obtenus peuvent être schématisés ainsi, les boules blanches correspondant au styrène alors que les boules foncées correspondent à l'acide méthacrylique :

<u>10% en acide méthacrylique initialement:</u>

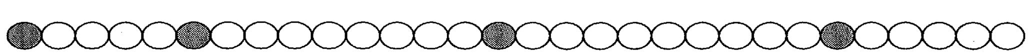

Copolymère à gradient très faible dont on ne peut attendre une nanostructuration.

20% en acide méthacrylique initialement:

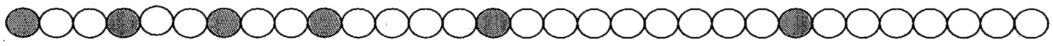

Copolymère à « tête » hydrophile et queue hydrophobe avec un gradient suffisamment prononcé pour conduire à une nanostructuration.

50% en acide méthacrylique initialement:

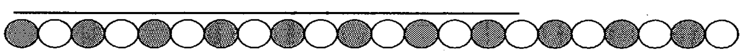

Les monomères étant isoréactifs dans ces conditions, le copolymère obtenu est du type alterné.

La structure de ces polymères peut être déterminée par la disparition de l'acide méthacrylique en fonction du taux de conversion.

Le copolymère à gradient selon l'invention comprend au moins deux monomères différents, qui peuvent être présents chacun à raison de 1 à 99% en poids par rapport au copolymère final, notamment à raison de 2-98% en poids, de préférence à raison de 5-95% en poids.

De préférence, le copolymère à gradient comprend au moins un monomère hydrophile, ou un mélange de monomères hydrophiles.

Ces monomères hydrophiles peuvent être présents à raison de 1 à 99% en poids, notamment 2-70% en poids, encore mieux 5-50% en poids, voire 10-30% en poids, par rapport au poids total du copolymère.

Dans la présente description, on désignera indifféremment par 'monomère hydrophile' les monomères dont les homopolymères sont solubles ou dispersibles dans l'eau, ou dont une forme ionique l'est.

Un homopolymère est dit hydrosoluble s'il forme une solution limpide lorsqu'il est en solution à 1 % en poids dans l'eau, à 25°C.

Un homopolymère est dit hydrodispersible si, à 1 % en poids dans l'eau, à 25°C, il forme une suspension stable de fines particules, généralement sphériques. La taille moyenne des particules constituant ladite dispersion est inférieure à 1 µm et, plus généralement, varie entre 5 et 400 nm, de préférence de 10 à 250 nm.

Ces tailles de particules sont mesurées par diffusion de lumière.

De préférence, le monomère hydrophile à une Tg supérieure ou égale à 20°C, notamment supérieure ou égale à 50°C, mais peut éventuellement avoir une Tg inférieur ou égale à 20°C.

Le copolymère à gradient peut également comprendre au moins un monomère hydrophobe, en particulier de monomère hydrophobe susceptible d'être rendu hydrophile après polymérisation, ou un mélange de tels monomères.

Le(s) monomère(s) hydrophobes peuvent être rendus hydrophile(s) par exemple par réaction chimique notamment par hydrolyse, ou par modification chimique, en particulier d'une fonction ester par incorporation de chaînes comportant un motif hydrophile, par exemple de type acide carboxylique.

Ces monomères hydrophobes peuvent être présents à raison de 1 à 99% en poids, notamment 30-98% en poids, encore mieux 50-95% en poids, voire 70-90% en poids, par rapport au poids total du copolymère.

Notamment, les monomères hydrophobes susceptibles d'être rendus hydrophiles peuvent être présents à raison de 1 à 99% en poids, notamment 5-50% en poids, et encore mieux 8-25% en poids, par rapport au poids total du copolymère.

De préférence, le monomère hydrophobe à une Tg supérieure ou égale à 20°C, notamment supérieure ou égale à 30°C, mais peut éventuellement avoir une Tg inférieur ou égale à 20°C.

De préférence, le copolymère à gradient selon l'invention comprend au moins un monomère ayant une Tg inférieure ou égale à 20°C, notamment comprise entre -150°C et 20°C, plus particulièrement comprise entre -130°C et 18°C, et encore mieux comprise entre -120°C et 15°C, ou un mélange de tels monomères.

Ces monomères de Tg $\leq$ 20°C peuvent être présents à raison de 1 à 99% en poids, notamment 10-90% en poids, encore mieux 20-80% en poids, voire 50-75% en poids, par rapport au poids total du copolymère.

Les monomères de Tg $\geq$ 20°C peuvent donc être présents à raison de 1 à 99% en poids, notamment 10-90% en poids, encore mieux 20-80% en poids, voire 25-50% en poids, par rapport au poids total du copolymère.

Dans la présente description, on désignera par 'monomère de Tg' les monomères dont l'homopolymère a une telle Tg, mesurée selon la méthode décrite ci-après.

Dans la présente invention, la Tg (ou température de transition vitreuse) est mesurée selon la norme ASTM D3418-97, par analyse enthalpique différentielle (DSC "Differential Scanning Calorimetry) sur calorimètre, sur une plage de température comprise entre -100°C et +150°C à une vitesse de chauffe de 10°C/min dans des creusets en

aluminium de 150 µl.

Dans un mode de réalisation préféré, le copolymère à gradient selon l'invention comprend trois monomères différents, qui peuvent être présents à raison de 5-90% en poids chacun, notamment 7-86% en poids chacun par rapport au poids total du copolymère.

En particulier, le copolymère peut comprendre 5-25% en poids d'un premier monomère, 5-25% en poids d'un second monomère et 50-90% en poids d'un troisième monomère.

Préférentiellement, le copolymère selon l'invention peut comprendre 5-25% en poids d'un monomère hydrophile, 50-90% en poids d'un monomère de Tg inférieure ou égale à 20°C et 5-25% en poids d'un monomère additionnel.

Parmi les monomères hydrophiles susceptibles d'être employés dans la présente invention, on peut citer les monomères suivants :

- les dérivés de (méth)acrylates d'aminoalkyles en C1-C6, et notamment les (méth)acrylates N,N-dialkyle en C1-C4 aminoalkyle en C1-C6 tels que le méthacrylate de N,N-diméthylaminoéthyle (MADAME) ou le méthacrylate de N, N-diéthylaminoéthyle (DEAMEA) ;
- les (méth)acrylamides N,N-dialkyle en C1-C4 éventuellement aminoalkyle en C1-C6, tels que la N,N-diméthylacrylamide, le N,N-diméthylaminopropylacrylamide (DMAPA) ou le N,N-diméthylaminopropylmethacrylamide (DMAPMA),
- les dialkyl dallylamines en C1-C8 tels que la diméthyldiallylamine;
- la vinylamine;
- les vinylpyridines et notamment la 2-vinylpyridine ou la 4-vinylpiridine ;

ainsi que leurs sels d'acides minéraux, ou d'acide organique, ou leurs formes quaternisées.

Parmi les acides minéraux, on peut citer l'acide sulfurique ou l'acide chlorhydrique, l'acide bromhydrique, iodhydrique, l'acide phosphorique, l'acide borique.

Parmi les acides organiques, on peut citer les acides comportant un ou plusieurs groupes carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyles.

Un exemple d'acide à groupe alkyle est l'acide acétique ou l'acide propionique. Un exemple de polyacide est l'acide téréphtalique. Des exemples d'hydroxyacides sont l'acide citrique et l'acide tartrique.

Les agents quaternisants peuvent être des halogénures d'alkyles tel que bromure de méthyle ou des sulfate d'alkyle tels le methylsulfate ou la propane sultone.

On peut encore citer :

- les acides carboxyliques, notamment mono- ou di-carboxyliques, éthyléniques tels que l'acide acrylique, l'acide méthacrylique, l'acide crotonique, l'acide itaconique, l'acide fumarique, l'acide maléique ;
- les anhydrides carboxyliques porteurs d'une liaison vinylique tels que l'anhydride maléique
- les acides sulfoniques éthyléniques tels que l'acide styrènesulfonique, l'acide acrylamidopropanesulfonique, et leurs sels;
- l'acide vinylbenzoïque, l'acide vinylphosphonique et leurs sels;
- le sel de potassium de l'acryloyloxy-3-sulfopropyle, ou le composé de formule $CH_2=CHCOOCH_2OCH_2(OH)CH_2SO_3^-Na^+$

Le neutralisant peut être une base minérale, telle que LiOH, NaOH, KOH, $Ca(OH)_2$, $NH_4OH$; ou une base organique, par exemple une amine primaire, secondaire ou tertiaire, notamment une alkylamine éventuellement hydroxylée comme la dibutylamine, la triéthylamine, la stearamine, ou bien l'amino-2-méthyl-2-propanol, la monoéthanolamine, la diéthanolamine, la stearamidopropyldimethylamine.

On peut encore citer :

- les amides des acides carboxyliques insaturés comme l'acrylamide ou le méthacrylamide et leur dérivés N-substitués tels que les (méth)acrylamides de N-alkyle en $C_1$-$C_4$ comme la N-méthylacrylamide; les (méth)acrylamides de N,N-dialkyle $C_1$-$C_4$, comme la N,N-diméthylacrylamide;
- les (méth)acrylates d'hydroxyalkyle notamment ceux dont le groupe alkyle a de 2 à 4 atomes de carbone, en particulier le (méth)acrylate d'hydroxyéthyle;
- les (méth)acrylates de polyéthylène glycol (5 à 100 OE) ou de glycol substitués ou non sur leur fonction terminale par des groupements alkyl, phosphates, phosphonates, sulfonate, par exemple l'acrylate de glycérol, le (méth) acrylate de methoxypolyethylene glycol (8 ou 12OE); le (méth)acrylate d'hydroxypolyethylene glycol ;
- les (méth)acrylates d'alkoxyalkyle tels que le (méth)acrylate d'éthoxyéthyle;

- les (méth)acrylates de polysaccharide comme l'acrylate de saccharose.
- les vinylamides telles que la vinyl acétamide; éventuellement cycliques en particulier les vinyllactames tels que la N-vinylpyrrolidone ou la N-vinylcaprolactame;
- les vinyléthers tels que le vinylmethyléther.

On peut encore citer :

- le methacrylamidopropoxy trimethylammoniumbetaine;
- le N,N-dimethyl-N-methacryloxyéthyl-N-(3-sulfopropyl)ammoniumbetaine,
- le 3-methacryloylethoxycarbonylpyridinium
- le composé de formule :

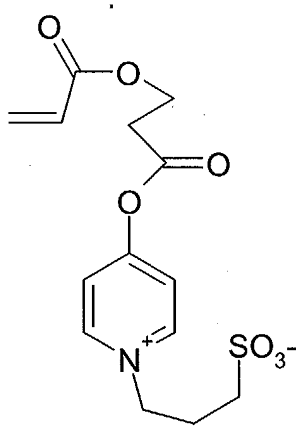

- le 4-vinylpyridyniumsulfopropylbetaine de formule :

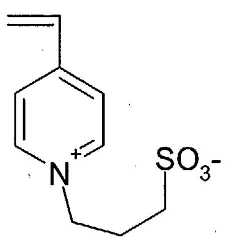

De préférence, les monomères hydrophiles sont choisis parmi le méthacrylate de N,N-diméthylaminoéthyle (MADAME), l'acide acrylique, l'acide méthacrylique, l'acide crotonique, l'acide styrènesulfonique, l'acide acrylamidopropanesulfonique, le diméthylaminopropylmethacrylamide (DMAPMA); le styrène sulfonate, l'acrylate d'hydroxyéthyle, l'acrylate de glycérol, le méthacrylate d'éthoxyéthyle, l'acrylate d'éthoxyéthyle, le (méth)acrylate de methoxypolyethylene glycol (8 ou 12OE); le (méth)acrylate d'hydroxypolyethylene glycol ; la N-vinylpyrrolidone, la N-vinylcaprolactame, l'acrylamide, la N,N-dimethylacrylamide.

Parmi les monomères hydrophobes susceptibles d'être rendus hydrophiles, notamment par hydrolyse, on peut citer les (méth)acrylates d'alkyles en C1-C4, tels que le (méth)acrylate de tertio-butyle ou le (méth)acrylate d'éthyle, qui conduisent à l'obtention de l'acide (méth)acrylique après hydrolyse.

Parmi les monomères dont l'homopolymère a une Tg inférieure ou égale à 20°C, et dont certains peuvent être hydrophiles, susceptibles d'être employés dans la présente invention, on peut citer :

- les hydrocarbures éthyléniques de 2 à 10 carbone, tels que l'éthylène, l'isoprène, et le butadiène ;
- les acrylates de formule CH2 = $CHCOOR_1$, $R_1$ représentant un groupe hydrocarboné saturé ou non, de 1 à 12 carbones, linéaire ou ramifié à l'exception du groupe tertio-butyle, dans lequel se trouve(nt) éventuellement intercalé(s) un ou plusieurs hétéroatomes choisis parmi O, N, S, Si, ledit groupe alkyle pouvant en outre être éventuellement substitué par un ou plusieurs substituants choisis parmi les groupes hydroxyle et les atomes d'halogène (Cl, Br, I et F) ;
  des exemples de groupes $R_1$ sont les groupes méthyle, éthyle, propyle, butyle, isobutyle, hexyle, éthylhexyle,

octyle, lauryle, isooctyle, isodécyle, hydroxyéthyle, hydroxypropyle, méthoxyéthyle, éthoxyéthyle, méthoxypropyle, éthylperfluorooctyle, et propylpolydiméthylsiloxane;
$R_1$ peut aussi être un groupement -(R")x-($OC_2H_4$)$_n$-OR"', avec x = 0 ou 1, R" = groupe hydrocarboné saturé ou non, de 1 à 12 carbones, linéaire ou ramifié, n = 5 à 100 et R"' = H ou $CH_3$; et notamment un groupement méthoxy (POE)8-stéaryle.

- les méthacrylates de formule : $CH_2$=C($CH_3$)-$COOR_2$
dans laquelle $R_2$ représente un groupe hydrocarboné saturé ou non, de 3 à 12 carbones, linéaire ou ramifié, dans lequel se trouve(nt) éventuellement intercalé(s) un ou plusieurs hétéroatomes choisis parmi O, N, S et Si, ledit groupe alkyle pouvant en outre être éventuellement substitué par un ou plusieurs substituants choisis parmi les groupes hydroxyle et les atomes d'halogènes (Cl, Br, I, F) ;
des exemples de groupes $R_2$ sont hexyle, éthylhexyle, octyle, lauryle, isooctyle, isodécyle, dodécyle, méthoxyéthyle, méthoxypropyle, éthoxyéthyle; éthylperfluorooctyle, et propylpolydiméthylsiloxane;
$R_2$ peut aussi être un groupement -(R")x-($OC_2H_4$)$_n$-OR"', avec x = 0 ou 1, R" = groupe hydrocarboné saturé ou non, de 1 à 12 carbones, linéaire ou ramifié, n = 5 à 100 et R"' = H ou $CH_3$; et notamment un groupement méthoxy (POE)8-stéaryle.
- les dérivés N ou N,N-substitués des amides d'acides carboxyliques insaturés en C1-12, notamment les (méth) acrylamides de N-alkyle en C1-12, tel que le N-octylacrylamide;
- les esters de vinyle de formule : $R_3$-CO-O-CH = $CH_2$ où $R_3$ représente un groupe alkyle de 2 à 12 carbone, linéaire ou ramifié, notamment le propionate de vinyle, le butyrate de vinyle, l'éthylhexanoate de vinyle, le néononanoate de vinyle, et le néododécanoate de vinyle ;
- les éthers de vinyle et d'alkyle ayant 1 à 12 carbones tels que l'éther de vinyle et de méthyle, et l'éther de vinyle et d'éthyle ;

De préférence, les monomères de Tg inférieure ou égale à 20°C sont choisis parmi :

- l'isoprène et le butadiène ;
- les acrylates de méthyle, d'éthyle, d'isobutyle, de n-butyle, d'éthylhexyle; de méthoxyéthyle, d'éthoxyéthyle; d'hydroxypolyéthylèneglycol;
- les méthacrylates d'éthoxyéthyle, d'hexyle, d'éthylhexyle; d'hydroxypolyéthylèneglycol;
- les (méth)acrylamides de N-alkyle en C6-12, tel que le N-octylacrylamide;
- les esters de vinyle de formule : $R_3$-CO-O-CH = $CH_2$ où $R_3$ représente un groupe alkyle de 6 à 12 carbones, linéaire ou ramifié, notamment le néononanoate de vinyle et le néododécanoate de vinyle.

Parmi les monomères dont l'homopolymère a une Tg supérieure ou égale à 20°C, et dont certains peuvent être hydrophiles, susceptibles d'être employés dans la présente invention, on peut citer :

- les composés vinyliques de formule : $CH_2$ = CH-$R_4$, où $R_4$ est un groupe hydroxyle ; un groupe -NH-C(O)-$CH_3$, un groupe -OC(O)-$CH_3$, un groupe cycloalkyle en $C_3$ à $C_8$ ; un groupe aryle en $C_6$ à $C_{20}$ ; un groupe aralkyle en $C_7$ à $C_{30}$ (groupe alkyle en $C_1$ à $C_4$) ; un groupe hétérocyclique de 4 à 12 chaînons contenant un ou plusieurs héréatomes choisis parmi O, N, et S ; un groupe hétérocyclylalkyle (alkyle en $C_1$ à $C_4$) tel qu'un groupe furfuryle ; lesdits groupes cycloalkyle, aryle, aralkyle, hétérocyclique, ou hétérocyclylalkyle pouvant être éventuellement substitué par un ou plusieurs substituants choisis parmi les groupes hydroxyles, les atomes d'halogène, et les groupes alkyles de 1 à 4 carbone, linéaires ou ramifiés dans lesquels se trouve(nt) éventuellement intercalé(s) un ou plusieurs hétéroatomes choisis parmi O, N, S et P, et lesdits groupes alkyle pouvant, en outre, être éventuellement substitués par un ou plusieurs substituants choisis parmi les groupes hydroxyles, et les atomes d'halogène (Cl, Br, I et F) ou Si.
Des exemples de monomères vinyliques sont le vinylcyclohexane, le styrène et l'acétate de vinyle.
- les acrylates de formule $CH_2$ = CH-$COOR_5$, où $R_5$ est un groupe tertiobutyle, un groupe cycloalkyle en $C_3$ à $C_8$ ; un groupe aryle en $C_6$ à $C_{20}$ ; un groupe aralkyle en $C_7$ à $C_{30}$ (groupe alkyle en $C_1$ à $C_4$) ; un groupe hétérocyclique de 4 à 12 chaînons contenant un ou plusieurs hétéroatomes choisis parmi O, N, et S ; un groupe hétérocyclylalkyle (alkyl de $C_1$ à $C_4$), tel qu'un groupe furfuryle ; lesdits groupes cycloalkyle, aryle, aralkyle, hétérocyclique ou hétérocyclylalkyle pouvant être éventuellement substitué par un ou plusieurs substituants choisis parmi les groupes hydroxyles, les atomes d'halogène, et les groupes alkyles de 1 à 4 C linéaires ou ramifiés dans lesquels se trouve (nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle et les atomes d'halogène (Cl, Br, I et F) ou Si. Des exemples de monomères acrylate sont les acrylates de t-butylcyclohexyle, de tertiobutyle, de t-butylbenzyle, de furfuryle et d'isobornyle ;
- les méthacrylates de formule $CH_2$ = C($CH_3$)-$COOR_6$, où $R_6$ est un groupe alkyle de 1 à 4C, linéaire ou ramifié, tel

qu'un groupe méthyle, éthyle, propyle ou isobutyle, ledit groupe alkyle pouvant en outre être éventuellement substitué par un ou plusieurs substituants choisis parmi les groupes hydroxyle et les atomes d'halogène (Cl, Br, I et F) ou Si; un groupe cycloalkyle en $C_3$ à $C_8$ ; un groupe aryle en $C_6$ à $C_{20}$ ; un groupe aralkyle en $C_7$ à $C_{30}$ (groupe alkyle en $C_1$ à $C_4$) ; un groupe hétérocyclique de 4 à 12 chaînons contenant un ou plusieurs hétéroatomes choisis parmi O, N, et S ; un groupe hétérocyclylalkyle (alkyle de 1 à 4 C), tel qu'un groupe furfuryle ; lesdits groupes cycloalkyle, aryle, aralkyle, ou hétérocyclique ou hétérocyclylalkyle pouvant être éventuellement substitué par un ou plusieurs substituants choisis parmi les groupes hydroxyles, les atomes d'halogène, et les groupes alkyles de 1 à 4 carbone, linéaires ou ramifiés, dans lequel se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle et les atomes d'halogène (Cl, Br, I et F).
Des exemples de monomères méthacrylate sont les méthacrylates de méthyle, d'éthyle, de n-butyle, d'isobutyle, de t-butylcyclohexyle, de t-butylbenzyle, de méthoxyéthyle de méthoxypropyle et d'isobornyle ;

- les (méth)acrylamides de formule : $CH_2$=C(R')-CO-$NR_7R_8$,

où $R_7$ et $R_8$ identiques ou différents représentent un atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle, et R' désigne H
ou méthyle.
Des exemples de monomères (méth)acrylamide sont le N-butylacrylamide, le N-t-butylacrylamide, le N-isopropylacrylamide, le N,N-diméthylacrylamide et le N,N-dibutylacrylamide.
Les monomères de Tg supérieure ou égale à 20°C préférés sont choisis parmi :

- les acrylates de furfuryle, d'isobornyle, de tertiobutyle, de tertiobutylecyclohexyle, de tertiobutylbenzyle;
- les méthacrylates de méthyle, de n-butyle, d'éthyle, d'isobutyle,
- le styrène, le styrène sulfonate;
- l'acétate de vinyle et le vinylcyclohexane.

L'homme du métier saura choisir les monomères et leurs quantités en fonction du résultat recherché, en se basant sur ses connaissances générales, notamment sur la réactivité relative de chaque monomère.
Ainsi, si l'on souhaite un copolymère ayant des motifs hydrophiles dans le coeur d'une chaîne polymère, on choisira préférentiellement un initiateur di-fonctionnel et un mélange de monomères tel que la réactivité des monomères hydrophiles est supérieure à celle des autres monomères.
Par ailleurs, on a constaté que les procédés de préparation employés permettaient d'ajuster et de moduler la ou les Tg du copolymère, et ainsi d'obtenir un copolymère à gradient ayant une ou plusieurs Tg donnée(s).
Les copolymères à gradient de l'invention peuvent être préparés par l'homme du métier suivant le mode opératoire suivant :

1/ On prépare un mélange des différents monomères, éventuellement dans un solvant, de préférence dans un réacteur et sous agitation. On ajoute un initiateur de polymérisation radicalaire et un agent de contrôle de la polymérisation. Le mélange est mis de préférence sous atmosphère de gaz inerte par rapport à une polymérisation radicalaire tel que l'azote ou l'argon.
Comme solvant éventuel de polymérisation, on peut choisir les acétates d'alkyle tels que l'acétate de butyle ou l'acétate d'éthyle, des solvants aromatiques tels que le toluène, des solvants cétoniques tels que la méthyléthylcétone, des alcools tels que l'éthanol. Dans le cas où le mélange de monomères est miscible à l'eau, celle ci peut être avantageusement utilisée comme solvant ou co-solvant.
2/ On porte le mélange sous agitation à la température de polymérisation souhaitée. Cette température est de préférence choisie dans une gamme allant de 10°C à 160°C, de préférence de 25°C à 130°C.
Le choix de la température de polymérisation est de préférence optimisé en fonction de la composition chimique du mélange de monomères. Ainsi, les monomères ayant des constantes cinétiques de propagation très élevée et une affinité pour l'agent de contrôle plus faible seront de préférence polymérisés à basse température (par exemple dans le cas d'une proportion importante de dérivés méthacryliques, on préfèrera une polymérisation à température comprise entre 25° et 80°C).
3/ Le milieu de polymérisation est éventuellement modifié pendant la polymérisation, avant d'atteindre 90% de conversion des monomère initiaux, par ajout supplémentaire d'un ou plusieurs monomères, notamment du mélange initial. Cet ajout peut être réalisé de différentes manières, pouvant aller de l'addition brutale en une seule fois à l'addition continue sur la durée totale de la polymérisation.
4/ La polymérisation est arrêtée lorsque le taux de conversion souhaité est atteint. De cette conversion dépend la composition globale du copolymère. De préférence, on arrête la polymérisation après avoir atteint au moins 50%

de conversion, notamment au moins 60%, préférentiellement après avoir atteint au moins 90% de conversion.
5/ Les éventuels monomères résiduels peuvent être éliminés par toute méthode connue, telle que par évaporation, ou par ajout d'une quantité d'initiateur classique de polymérisation tel que les dérivés peroxydiques ou azoïques.

Dans un premier mode de réalisation, l'agent de contrôle de la polymérisation susceptible d'être employé est un nitroxyde de formule (I), seul ou en mélange :

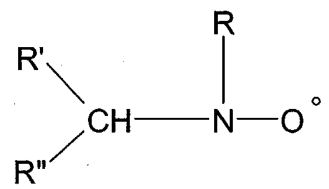
(I)

dans laquelle :

- R et R', indépendamment l'un de l'autre, sont des groupements hydrocarbonés saturés (alkyles) linéaires ou ramifiés, comportant 1 à 40 atomes de carbone, éventuellement substitués par un ou plusieurs groupements choisis parmi $-OR_3$, $-COOR_3$ et $-NHR_3$ (avec $R_3$ représentant H ou un groupement hydrocarboné saturé (alkyle) linéaire ou ramifié, comportant 1 à 40 atomes de carbone), R et R' pouvant en outre être reliés de manière à former un cycle; En particulier R et R' sont des groupements alkyle linéaires ou ramifiés comportant 1 à 12 atomes de carbone, notamment des groupements méthyle, éthyle, propyle, n-butyle, iso-butyle, ter-butyle, pentyle. De préférence, R et R' sont tous les deux des groupements ter-butyle.
- R" est un groupement monovalent de masse molaire (Mw) supérieure à 16 g/mol, notamment un groupement phosphoré de formule :

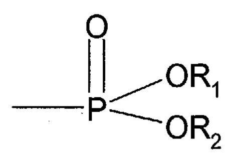

dans laquelle $R_1$ et $R_2$, indépendamment l'un de l'autre, sont des groupements hydrocarbonés saturés (alkyle) linéaires ou ramifiés, comportant 1 à 40 atomes de carbone, éventuellement substitués par un ou plusieurs groupements choisis parmi $-OR_3$, $-COOR_3$ et $-NHR_3$ (avec $R_3$ représentant H ou un groupement hydrocarboné saturé (alkyle) linéaire ou ramifié, comportant 1 à 40 atomes de carbone), $R_1$ et $R_2$ pouvant en outre être reliés de manière à former un cycle. En particulier $R_1$ et $R_2$ sont des groupements alkyle linéaires ou ramifiés comportant 1 à 12 atomes de carbone, notamment des groupements méthyle, éthyle, propyle, n-butyle, iso-butyle, ter-butyle, pentyle. De préférence, R1 et R2 sont tous les deux des groupements éthyle.

L'initiateur de polymérisation radicalaire peut être choisi parmi tous les initiateurs de polymérisation usuels, tels que les composés de type azoïque et notamment l'azobisisobutyronitrile, ou de type peroxyde tels que les peroxydes organiques ayant 6-30 atomes de carbone, notamment le peroxyde de benzoyle.
De préférence, on respecte un ratio molaire nitroxyde/initiateur compris entre 1 et 2,5; ce ratio peut être compris entre 2 et 2,5 lorsque l'on considère qu'une mole d'initiateur donne naissance à deux moles de chaînes polymères, et peut être compris entre 1 et 1,25 pour des initiateurs monofonctionnels.
Dans un second mode de réalisation particulier, on peut employer comme initiateur de polymérisation radicalaire des alcoxyamines de formule (II) qui peuvent être avantageusement choisies pour initier la polymérisation et libérer en même temps le nitroxyde contrôlant cette polymérisation.

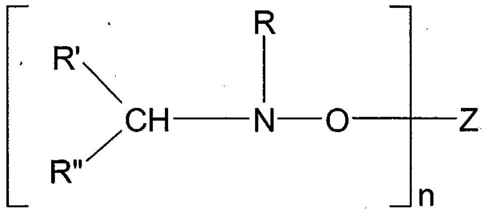

(II)

dans laquelle :

- R, R' et R" ont les significations données ci-dessus pour le nitroxyde de formule (I),
- n est un entier inférieur ou égal à 8, de préférence compris entre 1 et 3;
- Z est un radical monovalent ou multivalent, notamment un radical styryle, acryle ou méthacryle.

On peut également ajouter à l'alcoxyamine de formule (II), un nitroxyde de formule (I), dans une proportion allant de 0 à 20% molaires par rapport aux moles de fonctions alcoxyamines (une mole d'alcoxyamine multivalente apporte un nombre de fonctions alcoxyamine proportionnel à sa valence), de manière à améliorer la qualité du contrôle de polymérisation.
L'homme du métier saura choisir l'initiateur en fonction des besoins de l'application. Ainsi, un initiateur monofonctionnel conduira à des chaînes dissymétriques, alors que 'un initiateur poly-fonctionnel conduira à des macromolécules ayant une symétrie à partir d'un coeur.
Les copolymères à gradient selon l'invention peuvent être présents dans les compositions capillaires en une quantité de 0,1 à 60% en poids, de préférence 0,5 à 40% en poids, notamment 1 à 35% en poids, voire 5-30% en poids, par rapport au poids total de la composition.
Les copolymères peuvent être présents dans la composition sous forme solubilisée, par exemple dans l'eau ou un solvant organique, ou bien sous forme de dispersion aqueuse ou organique.
Il est possible de préparer une solution aqueuse du copolymère directement par mélange du polymère avec l'eau, éventuellement en chauffant.
On peut aussi dissoudre le polymère dans un solvant organique de température d'ébullition inférieur à l'eau (par exemple l'acétone ou la méthyléthylcétone), à un taux de solide compris entre 20 et 90% en poids.
Lorsque les monomères hydrophiles sont de type acide, on peut ajouter à la solution organique, une solution de préférence au moins 1 M de base, telle qu'un sel d'ion hydroxonium ($OH^-$), une amine (l'ammoniac), un sel de carbonate ($CO_3^{2-}$) ou d'hydrogénocarbonate ($HCO_3^-$) ou de neutralisant organique. Dans le cas de monomères hydrophiles de type amine, on peut ajouter une solution, de préférence au moins 1 M, d'acide. On ajoute alors l'eau à la solution sous vive agitation dans une proportion telle que le taux de solide obtenu soit compris entre 1 et 80% en poids. Eventuellement, l'eau peut être remplacée par un mélange hydroalcoolique, dans des proportions allant de 99/1 à 50/50. On évapore le solvant en agitant la solution à 100°C. La concentration se poursuit jusqu'à l'obtention du taux de solide désiré.
Un polymère est dit soluble lorsqu'il forme, à 1 % en poids, une solution limpide à 25°C.
Il est dit dispersible lorsqu'il forme, à 1 % en poids, à 25°C, une suspension stable de fines particules, généralement sphériques et dont la taille moyenne est de préférence inférieure à 1 micron, par exemple comprise entre 5 et 400 nm, de préférence 10 à 250 nm, mesurée par diffusion de la lumière.
Par ailleurs, selon un mode préféré de réalisation de l'invention, la composition selon l'invention est apte à former un film qui satisfait à au moins l'une des conditions suivantes :

- une déformation à la rupture $\varepsilon_r$ comprise entre 5% et 2500%, de préférence entre 10% et 2000% et encore mieux entre 15% et 1000%, et/ou
- un module d'Young compris entre 0,5 et 1200 MPa, de préférence entre 1 et 1000 MPa, préférentiellement entre 2 et 800 MPa, et/ou
- une recouvrance élastique instantanée $\varepsilon_i$ supérieure ou égale à 10%, de préférence supérieure ou égale à 25%, et mieux supérieure ou égale à 35%, notamment comprise entre 10-100%, par exemple 25-98%, voire 35-95%.

On mesure les propriétés mécaniques du film en traction monotone selon la norme ASTM Standards, volume 06.01 D 2370-92 'Standard Test Method for Tensile Properties of Organic Coatings'.
On découpe une éprouvette dans le film libre obtenu par séchage, à 23 ± 2°C, à un taux d'humidité relative de 55 % ± 5 %, après un temps de séchage de 48 heures, d'une solution du copolymère dans un solvant approprié, par exemple

l'eau, la quantité de solution étant adaptée pour obtenir dans une matrice en téflon, un film ayant une épaisseur de 150 ± 50 µm.
L'éprouvette est de forme haltère, de longueur utile 33 mm et de largeur utile 6 mm. La section (s) de l'éprouvette est alors définie comme : s=largeur x épaisseur ($mm^2$).
Les essais sont réalisés sur un appareil de traction équipé d'un extensomètre optique pour la mesure du déplacement et commercialisé sous l'appellation Zwick Z010.
Les mesures sont réalisées à une température de 23 ± 2°C et une humidité relative de 55 ± 5%.
Les éprouvettes sont étirées à une vitesse de déplacement de 50 mm/minute. On impose donc une vitesse de déplacement et on mesure simultanément la longueur (L) de l'éprouvette et la force (f) nécessaire pour imposer cette longueur.
La longueur (L) est mesurée avec un extensomètre optique à l'aide de pastilles adhésives placées sur l'éprouvette haltère. La distance initiale entre ces 2 pastilles définie la longueur utile Lo utilisée pour calculer la déformation :

$$\varepsilon = (L/Lo) \times 100 \text{ exprimée en \%.}$$

Il est ainsi obtenu une courbe contrainte $\sigma$ (= f/s) en fonction de la déformation $\varepsilon$, l'essai étant conduit jusqu'à rupture de l'éprouvette. La déformation à la rupture $\varepsilon_r$ correspond à la déformation maximale de l'échantillon avant le point de rupture.
Le module d'Young (module d'élasticité), exprimé en MPa, correspond à la pente de la courbe $\sigma = f(\varepsilon)$, considérée dans la partie linéaire de la courbe (début de l'essai).
La recouvrance élastique (R donnée en %) est déterminée en étirant les éprouvettes à une vitesse de déplacement de 50 mm/minute jusqu'à une déformation de 100%, c'est-à-dire jusqu'à 2 fois sa longueur initiale. On relâche alors la contrainte en imposant une vitesse de retour égale à la vitesse de traction, soit 50 mm/minute.
La recouvrance élastique instantanée ($R_i = \varepsilon_i$) est déterminée par mesure de l'allongement de l'éprouvette (exprimé en % par rapport à la longueur initiale) immédiatement après retour à charge nulle. Elle est donc définie par la déformation instantanée résiduelle, à contrainte nulle :$\varepsilon_{iR}$.
La recouvrance élastique instantanée ($R_i = \varepsilon_i$) est définie par $R_i = \varepsilon_i = 100 - \varepsilon_{iR}$
Les compositions cosmétiques selon l'invention comprennent, outre lesdits copolymères, un milieu cosmétiquement acceptable, c'est-à-dire un milieu compatible avec les matières kératiniques et notamment avec les cheveux. Ce milieu peut être constitué d'eau ou d'un ou plusieurs solvants organiques cosmétiquement acceptables, tels que par exemple les alcools en $C_{1-4}$ en particulier l'éthanol, l'isopropanol, le tertio-butanol et le n-butanol, et les alkylèneglycols comme le propylèneglycol, ou encore un mélange d'eau et d'un ou de plusieurs solvants organiques cosmétiquement acceptables.
La composition du milieu cosmétiquement acceptable et sa proportion dans la composition capillaire finale sont de préférence telles que la teneur globale de la composition capillaire en composés organiques volatils (VOC ou volatil organic compounds en anglais) est de préférence inférieure ou égale à 55% en poids, notamment inférieure ou égale à 35% en poids, voire égale à 0%.
L'eau ou le mélange d'eau et de solvants organiques hydrophiles peut donc être présent dans la composition selon l'invention en une teneur allant de 0,1 à 99% en poids, par rapport au poids total de la composition, et de préférence de 10 à 80% en poids.
Les compositions capillaires de la présente invention peuvent comprendre en outre un ou plusieurs ingrédients cosmétiques ou de formulation, utilisés habituellement dans le domaine cosmétique.
On peut citer à titre d'exemples de tels ingrédients les polymères filmogènes anioniques, cationiques, non-ioniques ou amphotères; les polymères fixants; les silicones volatiles ou non volatiles; les agents tensioactifs anionique, cationiques, amphotères ou non-ioniques, les agents épaississants, les agents nacrants, les filtres UV, les agents antiradicalaires, les parfums, les agents conservateurs, les pigments et colorants, les agents d'ajustement du pH, les agents solubilisants, les agents plastifiants, les agents anti-mousse, les cires et huiles, les vitamines, des agents conditionneurs et les particules organiques ou minérales, synthétiques ou d'origine naturelle.
Bien entendu l'homme du métier veillera à choisir ce ou ces éventuels additifs complémentaires et/ou leurs quantités de manière à ce que les propriétés avantageuses intrinsèques des compositions de l'invention ne soient pas altérées par la ou les adjonctions envisagées.
Les polymères filmogènes fixants susceptibles d'être utilisés dans les compositions de la présente invention peuvent être d'origine naturelle, et éventuellement être modifiés, ou d'origine synthétique. Ils peuvent être choisis parmi les polymères fixants cationiques, anioniques, non-ioniques ou amphotères.
Parmi les polymères fixants cationiques, on peut citer les polymères comportant des groupements amine primaire, secondaire, tertiaire et/ou quaternaire faisant partie de la chaîne polymère ou directement reliés à celle-ci, et ayant une masse moléculaire moyenne en nombre comprise entre 500 et environ 5 000 000, et de préférence entre 1 000

et 3 000 000, tels que :

(1) les homopolymères ou copolymères dérivés d'esters ou d'amides acryliques ou méthacryliques et comportant des motifs correspondant à au moins une des formules suivantes :

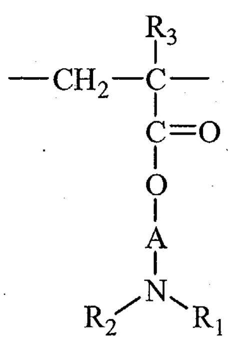

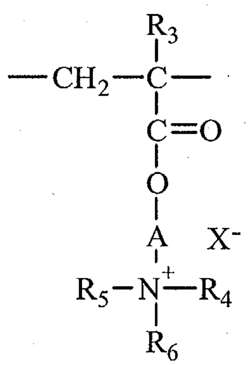

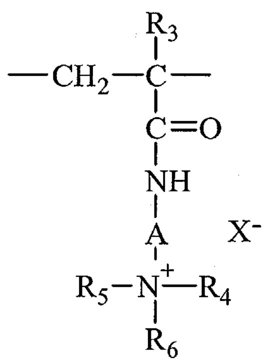

dans lesquelles:

$R_1$ et $R_2$ représentent chacun indépendamment un atome d'hydrogène ou un groupe alkyle en $C_{1-6}$ ;
$R_3$ désigne un atome d'hydrogène ou un groupe $CH_3$ ;
A est un groupe alkyle linéaire ou ramifié, comportant de 1 à 6 atomes de carbone ou un groupe hydroxyalkyle comportant de 1 à 4 atomes de carbone ;
$R_4$, $R_5$, $R_6$ représentent indépendamment chacun un groupe alkyle ayant de 1 à 18 atomes de carbone ou un groupe benzyle ;
X désigne un anion méthosulfate ou halogénure tel que chlorure ou bromure.

Les copolymères de la famille (1) contiennent en outre un ou plusieurs motifs dérivant de co-monomères choisis dans la famille des acrylamides, méthacrylamides, diacétone-acrylamides, acrylamides et méthacrylamides substitués sur l'azote par des groupes alkyle inférieur, des acides (méth)acryliques ou de leurs esters, vinyllactames tels que la vinylpyrrolidone ou le vinylcaprolactame, esters vinyliques.
Ainsi, parmi ces copolymères de la famille (1), on peut citer :

- les copolymères d'acrylamide et de méthacrylate de diméthylaminoéthyle quaternisés au sulfate de diméthyle ou avec un halogénure de méthyle tels que celui vendu sous la dénomination HERCOFLOC® par la société HERCULES,
- les copolymères d'acrylamide et de chlorure de méthacryloyloxyéthyltriméthylammonium décrits, par exemple, dans la demande de brevet EP-A-080976 et vendus sous la dénomination BINA QUAT® P100 par la société CIBA GEIGY,
- le copolymère d'acrylamide et de méthosulfate de méthacryloyloxyéthyltriméthylammonium vendu sous la dénomination RETEN® vendu par la société HERCULES,
- les copolymères vinylpyrrolidone/acrylate ou méthacrylate de dialkylaminoalkyle quaternisés ou non, tels que les produits vendus sous la dénomination GAFQUAT® par la société ISP comme, par exemple, GAFQUAT® 734 ou GAFQUAT® 755, ou bien les produits dénommés "COPOLYMER® 845, 958 et 937". Ces polymères sont décrits en détail dans les brevets français FR 2 077 143 et FR 2 393 573,
- les terpolymères méthacrylate de diméthylaminoéthyle/ vinylcaprolactame/vinylpyrrolidone tels que le produit commercialisé sous la dénomination GAFFIX® VC 713 par la société ISP, et
- les copolymères vinylpyrrolidone/méthacrylamide de diméthylaminopropyle quaternisé tels que notamment le produit commercialisé sous la dénomination GAFQUAT® HS 100 par la société ISP.

(2) les polysaccharides quaternisés décrits plus particulièrement dans les brevets américains US 3,589,578 et US 4,031,307 tels que les gommes de guar contenant des groupements cationiques trialkylammonium. De tels produits sont commercialisés notamment sous les dénominations JAGUAR C13 S, JAGUAR C 15, et JAGUAR C 17 par la société MEYHALL.

(3) les copolymères quaternaires de vinylpyrrolidone et de vinylimidazole, tels que les produits commercialisés

par la société BASF sous la dénomination LUVIQUAT TFC.

(4) les chitosanes et leurs sels tels que l'acétate, le lactate, le glutamate, le gluconate ou le pyrrolidone-carboxylate de chitosane.
Parmi ces composés, on peut citer le chitosane ayant un taux de désacétylation de 90,5 % en poids commercialisé sous la dénomination KYTAN BRUT STANDARD par la société ABER TECHNOLOGIES, le pyrrolidone-carboxylate de chitosane vendu sous la dénomination KYTAMER® PC par la société AMERCHOL.

(5) les dérivés de cellulose cationiques tels que les copolymères de cellulose ou de dérivés de cellulose greffés avec un monomère hydrosoluble comportant un ammonium quaternaire, et décrits notamment dans le brevet US 4,131,576, tels que les hydroxyalkylcelluloses, comme les hydroxyméthylcelluloses, les hydroxyéthylcelluloses et hydroxypropyl-celluloses greffées notamment avec un sel de méthacryloyloxyéthyl-tri-méthylammonium, de méthacrylamidoproyltriméthylammonium ou de diméthyldiallylammonium.
Ces polymères sont vendus notamment sous les dénominations CELQUAT® L200 et CELQUAT® H100 par la société NATIONAL STARCH.

Parmi les polymères fixants anioniques, on peut citer ceux comportant des groupements dérivés d'acide carboxylique, d'acide sulfonique ou d'acide phosphorique et ont une masse moléculaire moyenne en nombre comprise entre environ 500 et 5 000 000.
Les groupements carboxyliques sont apportés par des monomères mono- ou diacides carboxyliques insaturés tels que ceux répondant à la formule :

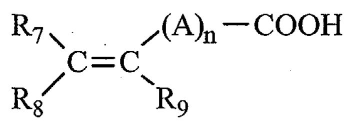

dans laquelle
n est un nombre entier de 0 à 10,
$A_1$ désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre,
$R_7$ désigne un atome d'hydrogène, un groupement phényle ou benzyle,
$R_8$ désigne un atome d'hydrogène, un groupement alkyle inférieur ou carboxyle, et
$R_9$ désigne un atome d'hydrogène, un groupement alkyle inférieur, un groupement $-CH_2-COOH$, phényle ou benzyle.
Dans la formule précitée, un groupement alkyle inférieur désigne de préférence un groupement ayant 1 à 4 atomes de carbone et en particulier, les groupements méthyle et éthyle.
Les polymères fixants anioniques à groupements carboxyliques préférés selon l'invention sont :

A) Les homo- ou copolymères d'acide acrylique ou méthacrylique ou leurs sels et en particulier les produits vendus sous les dénominations VERSICOL® E ou K par la société ALLIED COLLOID et ULTRAHOLD® par la société BASF, les copolymères d'acide acrylique et d'acrylamide vendus sous la forme de sel de sodium sous les dénominations RETEN® 421, 423 ou 425 par la société HERCULES, et les sels de sodium des acides polyhydroxycarboxyliques.
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylèneglycol tel que le polyéthylène-glycol, et éventuellement réticulés. De tels polymères sont décrits en particulier dans le brevet français FR 1 222 944 et la demande de brevet allemand DE 2 330 956, les copolymères de ce type comportant dans leur chaîne un motif acrylamide éventuellement N-alkylé et/ou N-hydroxyalkylé tels que décrits notamment dans les demandes de brevets luxembourgeois n° 75370 et 75371 et proposés sous la dénomination QUADRAMER® par la société AMERICAN CYANAMID. On peut également citer les copolymères d'acide acrylique et de méthacrylate d'alkyle en $C_1$-$C_4$ et les terpolymères de vinylpyrrolidone, d'acide acrylique et de méthacrylate d'alkyle en $C_1$-$C_{20}$, par exemple, de lauryle, tels que celui commercialisé par la société ISP sous la dénomination ACRYLIDONE® LM et les terpolymères acide méthacrylique/acrylate d'éthyle/acrylate de tertiobutyle tels que le produit commercialisé sous la dénomination LUVIMER® 100 P par la société BASF.
C) Les copolymères dérivés d'acide crotonique, tels que ceux comportant dans leur chaîne des motifs acétate ou propionate de vinyle, et éventuellement d'autres monomères tels que les esters allylique ou méthallylique, éther

vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés et réticulés, ou encore un autre monomère ester vinylique, allylique ou méthallylique d'un acide carboxylique □- ou □-cyclique. De tels polymères sont décrits entre autres dans les brevets français FR 1 222 944, FR 1 580 545, FR 2 265 782, FR 2 265 781, FR 1 564 110 et FR 2 439 798. Des produits commerciaux entrant dans cette classe sont les résines 28-29-30, 26-13-14 et 28-13-10 commercialisée par la société NATIONAL STARCH.

D) Les copolymères dérivés d'acides ou d'anhydrides carboxyliques monoinsaturés en $C_4$-$C_8$ choisis parmi :

- les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US n° 2,047,398, 2,723,248 et 2,102,113, et le brevet GB 839 805. Des produits commerciaux sont notamment ceux vendus sous les dénominations GANTREZ® AN ou ES par la société ISP.
- les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, □-oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées.

Ces polymères sont par exemple décrits dans les brevets français FR 2 350 384 et FR 2 357 241 de la demanderesse.

E) Les polyacrylamides comportant des groupements carboxylates.

Les polymères comprenant les groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :

- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone.
- les sels de l'acide polystyrène-sulfonique tels que les sels de sodium vendus par exemple sous les dénominations FLEXAN® 130 et FLEXAN® 500 par la société NATIONAL STARCH. Ces composés sont décrits dans le brevet FR 2 198 719.
- les sels d'acides polyacrylamide-sulfoniques tels que ceux mentionnés dans le brevet US 4,128,631, et plus particulièrement l'acide polyacrylamidoéthylpropanesulfonique vendu sous la dénomination COSMEDIA POLYMER® HSP 1180 par la société HENKEL.

Parmi les polymères fixants amphotères, on peut citer ceux comportant des motifs B et C répartis statistiquement dans la chaîne polymère où B désigne un motif dérivant d'un monomère comportant au moins un atome d'azote basique et C désigne un motif dérivant d'un monomère comportant un ou plusieurs groupements carboxyliques ou sulfoniques. Les polymères fixants amphotères peuvent également comporter des motifs zwitterioniques de type carboxybétaïne ou sulfobétaïne.

Il peut également s'agir de polymères à chaîne principale cationique comportant des groupements amine primaire, secondaire, tertiaire ou quaternaire, parmi lesquels au moins un porte, par l'intermédiaire d'un radical hydrocarboné, un groupement acide carboxylique ou acide sulfonique. Les polymères fixants amphotères peuvent encore avoir une chaîne anionique dérivée d'acides carboxyliques αβ-insaturés dont l'un des groupements carboxyle a été amené à réagir avec une polyamine comportant un ou plusieurs groupements amine primaire.

Les polymères fixants amphotères répondant à la définition donnée ci-dessus sont choisis notamment parmi les polymères suivants :

(1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alphachloroacrylique, et d'un monomère basique dérivé d'un composé vinylique substitué contenant au moins un atome basique, tel que plus particulièrement les méthacrylate et acrylate de dialkylaminoalkyle, les dialkylaminoalkylméthacrylamide et acrylamide. De tels composés sont décrits dans le brevet américain US 3,836,537.

(2) les polymères comportant des motifs dérivant :

(a) d'au moins un monomère choisi parmi les acrylamides ou les méthacrylamides substitués sur l'atome d'azote par un groupe alkyle,

(b) d'au moins un comonomère acide contenant un ou plusieurs groupements carboxyliques réactifs, et '

(c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.

Les acrylamides ou méthacrylamides N-substitués sont notamment les composés dont les groupes alkyle comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.

Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.

Les comonomères basiques préférés sont des méthacrylates d'aminoéthyle, de butylaminoéthyle, de N,N'-diméthylaminoéthyle, de N-tertio-butylaminoéthyle.

On utilise particulièrement les copolymères dont la dénomination CTFA ($4^{ème}$ Ed., 1991) est Octylacrylamide/acrylates/butylaminoethyl-methacrylate copolymer, tels que les produits vendus sous la dénomination AMPHOMER® ou LOVOCRYL® 47 par la société NATIONAL STARCH.

(3) les polyaminoamides réticulés et alcoylés partiellement ou totalement dérivant de polyaminoamides de formule générale :

$$-(CO-R_{10}-CO-Z-)-$$

dans laquelle $R_{10}$ représente un groupe divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides

ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :

dans les proportions de 60 à 100 % en moles, le groupe

$$-NH-[(CH_2)_x-NH]_p-$$

où x = 2 et p = 2 ou 3, ou bien x = 3 et p = 2

ce groupe dérivant de la diéthylène-triamine, de la triéthylène-tétraamine ou de la dipropylène-triamine;

dans les proportions de 0 à 40 % en moles, le groupe

$$-NH-[(CH_2)_x-NH]_p-$$

où x = 2 et p = 1 et qui dérive de l'éthylène-diamine, ou le groupe dérivant de la pipérazine :

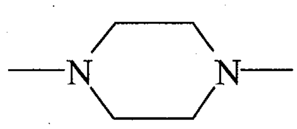

dans les proportions de 0 à 20 % en moles, le groupe

$$-NH-(CH_2)_6-NH-$$

dérivant de l'hexaméthylènediamine,

ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et alcoylés par action d'acide acrylique, d'acide chloracétique ou d'une alcane-sultone ou de leurs sels.
Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.
Les alcane-sultones utilisées dans l'alcoylation sont de préférence la propane- ou la butane-sultone, les sels des agents d'alcoylation sont de préférence les sels de sodium ou de potassium.

(4) les polymères comportant des motifs zwittérioniques de formule :

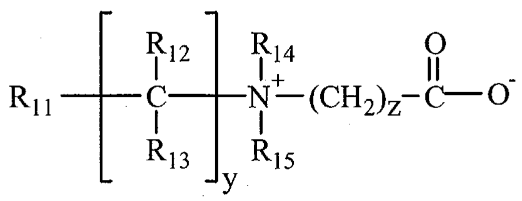

dans laquelle $R_{11}$ désigne un groupement insaturé polymérisable tel qu'un groupement acrylate, méthacrylate, acrylamide ou méthacrylamide, y et z représentent chacun un nombre entier de 1 à 3, $R_{12}$ et $R_{13}$ représentent un atome d'hydrogène, un groupe méthyle, éthyle ou propyle, $R_{14}$ et $R_{15}$ représentent un atome d'hydrogène ou un groupe alkyle de telle façon que la somme des atomes de carbone dans $R_{14}$ et $R_{15}$ ne dépasse pas 10.
Les polymères comprenant de telles unités peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.
A titre d'exemple, on peut citer les copolymères méthacrylate de méthyle/diméthylcarboxyméthylammonioéthylméthacrylate de méthyle, tels que le produit vendu sous la dénomination DIAFORMER® Z301 par la société SANDOZ.

(5) les polymères dérivés du chitosane comportant des motifs monomères répondant aux formules suivantes :

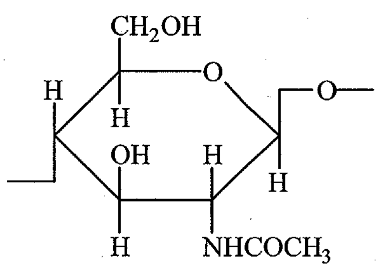

(A)

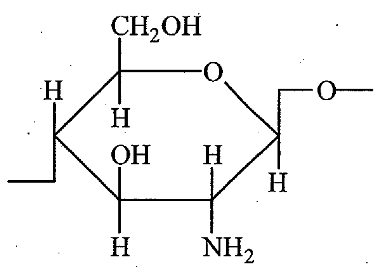

(B)

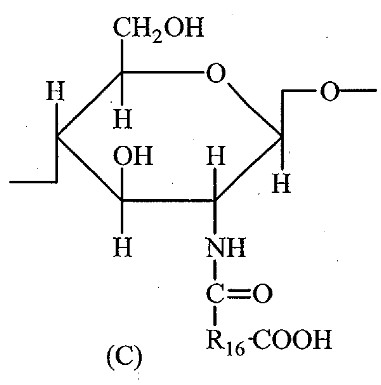

(C)

le motif (A) étant présent dans des proportions comprises entre 0 et 30%, le motif (B) dans des proportions comprises entre 5 et 50% et le motif (C) dans des proportions comprises entre 30 et 90%, étant entendu que dans ce motif (C), $R_{16}$ représente un groupe de formule :

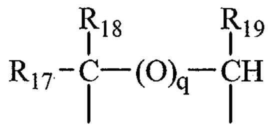

dans laquelle si q = 0, $R_{17}$, $R_{18}$ et $R_{19}$, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes $R_{17}$, $R_{18}$ et $R_{19}$ étant dans ce cas un atome d'hydrogène ;
ou si q = 1, $R_{17}$, $R_{18}$ et $R_{19}$ représentent chacun un atome d'hydrogène, ainsi que les sels formés par ces composés avec des bases ou des acides.

(6) Les polymères obtenus par N-carboxylation du chitosane tels que le N-carboxyméthylchitosane ou le N-carboxybutylchitosane vendu sous la dénomination EVALSAN® par la société JAN DEKKER.

(7) Les polymères décrits dans le brevet français FR 1 400 366 et répondant à la formule

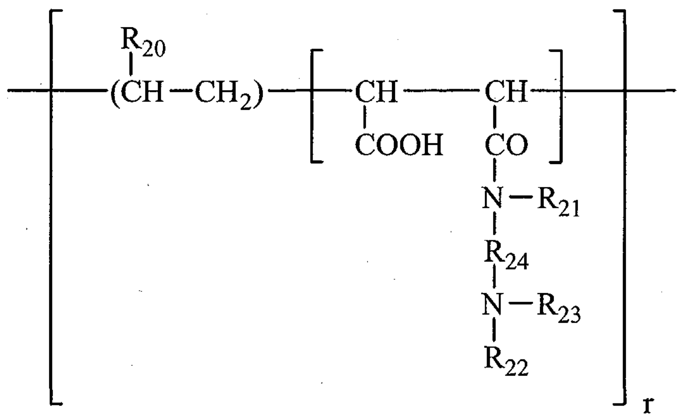

dans laquelle $R_{20}$ représente un atome d'hydrogène ou un groupe $CH_3O$, $CH_3CH_2O$ ou phényle, $R_{21}$ désigne un atome d'hydrogène ou un groupe alkyle inférieur tel que méthyle et éthyle, $R_{22}$ désigne un atome d'hydrogène ou un groupe alkyle inférieur en $C_1$-$C_6$ tel que méthyle et éthyle, $R_{23}$ désigne un groupe alkyle inférieur en $C_1$-$C_6$ tel que méthyle, éthyle ou un groupe répondant à la formule : $-R_{24}-N(R_{22})_2$, $R_{24}$ représentant un groupement $-CH_2-CH_2-$, $-CH_2-CH_2-CH_2-$, $-CH_2-CH(CH_3)-$, et $R_{22}$ ayant les significations mentionnées ci-dessus.

(8) Les polymères amphotères du type -D-X-D-X choisis parmi:

(a) les polymères obtenus par action de l'acide chloroacétique ou le chloroacétate de sodium sur les composés comportant au moins un motif de formule :

-D-X-D-X-D-

où D désigne un groupe

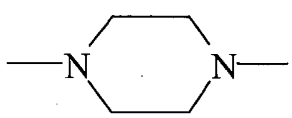

et X désigne le symbole E ou E', E ou E' identiques ou différents désignent un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.

(b) Les polymères de formule :

-D-X-D-X-

où D désigne un groupe

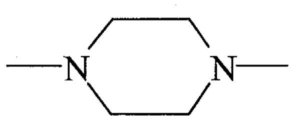

et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloroacétate de soude.

(9) les copolymères alkyl($C_1$-$C_5$)vinyléther/anhydride maléique modifiés partiellement par semi-amidification avec une N,N-dialkylaminoalkylamine telle que la N,N-diméthyaminopropylamine ou par semi-estérification avec une N,N-dialcanolamine. Ces copolymères peuvent également comporter d'autres comonomères vinyliques tels que le vinylcaprolactame.

Les polymères fixants non ioniques sont choisis, par exemple, parmi :

la vinylpyrrolidone,
les copolymères de vinylpyrrolidone et d'acétate de vinyle,
les polyalkyloxazolines telles que les polyéthyloxazolines proposées par la société DOW CHEMICAL sous les dénominations PEOX® 50 000, PEOX® 200 000 et PEOX® 500 000,
les homopolymères d'acétate de vinyle tels que le produit proposé sous la dénomination APPRETAN® EM par la société HOECHST ou le produit proposé sous le nom de RHODOPAS® A 012 par la société RHONE POULENC,
les copolymères d'acétate de vinyle et d'ester acrylique tels que le produit proposé sous le nom de RHODOPAS® AD 310 de la société RHONE POULENC,
les copolymères d'acétate de vinyle et d'éthylène tels que le produit proposé sous le nom de APPRETAN® TV par la société HOECHST,
les copolymères d'acétate de vinyle et d'ester maléique, par exemple, de maléate de dibutyle tels que le produit proposé sous le nom de APPRETAN® MB EXTRA par la société HOECHST,
les copolymères de polyéthylène et d'anhydride maléique,
les poly(acrylate d'alkyle) et poly(méthacrylate d'alkyle) tels que le produit proposé sous la dénomination MICROPEARL® RQ 750 par la société MATSUMOTO ou le produit proposé sous la dénomination LUHYDRAN® A 848 S par la société BASF,
les copolymères d'esters acryliques tels que, par exemple, les copolymères d'acrylates d'alkyle et de méthacrylates d'alkyle tels que les produits proposés par la société ROHM & HAAS sous les dénominations PRIMAL® AC-261 K et EUDRAGIT® NE 30 D, par la société BASF sous les dénominations ACRONAL® 601, LUHYDRAN® LR 8833 ou 8845, par la société HOECHST sous les dénominations APPRETAN® N9212 et N9213 ;
les copolymères d'acrylonitrile et d'un monomère non ionique choisis, par exemple, parmi le butadiène et les (méth) acrylates d'alkyle ; on peut citer les produits proposés sous les dénominations NIPOL® LX 531 B par la société NIPPON ZEON ou ceux proposés sous la dénomination CJ 0601 B par la société ROHM & HAAS,

les polyuréthannes tels que les produits proposés sous les dénominations ACRYSOL® RM 1020 ou ACRYSOL® RM 2020 par la société ROHM & HAAS, les produits URAFLEX® XP 401 et URAFLEX® XP 402 UZ par la société DSM RESINS,
les polyamides tels que le produit ESTAPOR® LO 11 proposé par la société RHONE POULENC,
les gommes de guar non ioniques chimiquement modifiées ou non modifiées. Les gommes de guar non modifiées sont par exemple les produits vendus sous la dénomination VIDOGUM® GH 175 par la société UNIPECTINE et sous la dénomination JAGUAR® C par la société MEYHALL. Les gommes de guar modifiées sont de préférence modifiées par des groupements hydroxyalkyle en $C_{1-6}$, de préférence par des groupements hydroxyméthyle, hydroxyéthyle, hydroxypropyle et hydroxybutyle. De telles gommes de guar non ioniques éventuellement modifiées par des groupes hydroxyalkyle sont par exemple vendues sous les dénominations commerciales JAGUAR® HP8, JAGUAR® HP60, JAGUAR® HP120, JAGUAR® DC 293 et JAGUAR® HP 105 par la société MEYHALL, ou sous la dénomination GALACTOSOL® 4H4FD2 par la société AQUALON.

On peut également utiliser, en tant que polymères fixants, des polymères filmogènes de type silicone greffée comprenant une partie polysiloxane et une partie constituée d'une chaîne organique non siliconée, l'une des deux parties constituant la chaîne principale du polymère et l'autre étant greffée sur ladite chaîne principale.
Ces polymères sont par exemple décrits dans les demandes de brevet EP-A-0 412 704, EP-A-0 412 707, EP-A-0 640 105 et WO 95/00578, EP-A-0582 152 et WO 93/23009 et les brevets US 4,693,935, US 4,728,571 et US 4,972,037.
Ces polymères sont de préférence anioniques ou non ioniques.
De tels polymères sont par exemple les copolymères susceptibles d'être obtenus par polymérisation radicalaire à partir du mélange de monomères formé

a) de 50 à 90 % en poids d'acrylate de tertiobutyle,
b) de 0 à 40 % en poids d'acide acrylique,
c) de 5 à 40 % en poids d'un macromère siliconé de formule

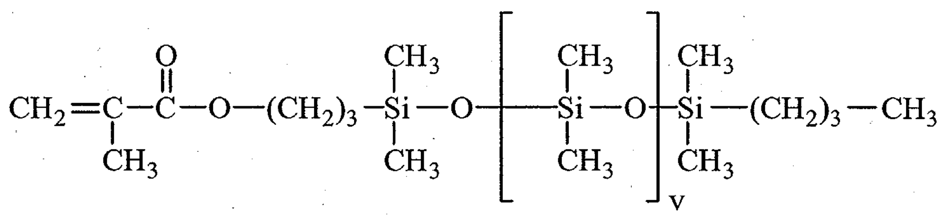

où v est un nombre allant de 5 à 700, les pourcentages en poids étant calculés par rapport au poids total des monomères.
D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.
On peut également utiliser des polymères contenant des motifs uréthanne. Ces polyuréthannes peuvent être fonctionnalisés ou non, siliconés ou non. Les polyuréthannes particulièrement visés sont ceux décrits dans les demandes EP 0 751 162, EP 0 637 600, EP 0 648 485 et FR 2 743 297 dont la demanderesse est titulaire, ainsi que dans les demandes EP 0 656 021 et WO 94/03510 de la société BASF, et dans les demandes EP 0 619 111 de la société National Starch. Comme polyuréthannes convenant particulièrement bien à la présente invention, on peut citer les produits commercialisés sous les dénominations LUVISETPUR® et LUVISET® Si PUR par la société BASF.
Le ou les polymères filmogènes fixants utilisés dans les compositions de la présente invention sont de préférence présents à raison de 0,05 à 10 % en poids rapporté au poids total de la composition.
Lorsque la composition est conditionnée dans un dispositif aérosol, elle comprend par ailleurs un agent propulseur qui peut être n'importe quel gaz liquéfiable utilisé habituellement dans des dispositifs aérosol tels que le diméthyléther, les alcanes en $C_{3-5}$, le 1,1-difluoroéthane, les mélanges de diméthyléther et d'alcanes en $C_{3-5}$, et les mélanges de 1,1-difluoroéthane et de diméthyléther et/ou d'alcanes en $C_{3-5}$.
On préfère tout particulièrement utiliser comme agent propulseur le diméthyléther et les alcanes en $C_{3-5}$, et en particulier le propane, le n-butane et l'isobutane.
L'agent propulseur est de préférence présent à raison de 35 à 70% du poids total du mélange (agent propulseur +

composition coiffante ) contenu dans le dispositif aérosol.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition selon l'invention peut être un produit capillaire, notamment pour le maintien de la coiffure ou la mise en forme des cheveux. Les compositions capillaires sont de préférence des shampooings, des gels, des lotions de mise en plis, des lotions pour le brushing, des compositions de fixation et de coiffage telles que les laques ou spray. Les lotions peuvent être conditionnées sous diverses formes, notamment dans des vaporisateurs, des flacons-pompe ou dans des récipients aérosol afin d'assurer une application de la composition sous forme vaporisée ou sous forme de mousse. De telles formes de conditionnement sont indiquées, par exemple lorsque l'on souhaite obtenir un spray, une mousse pour la fixation ou le traitement des cheveux.

Les compositions capillaires sont de préférence des laques pour cheveux ou des mousses.

L'invention est illustrée plus en détail dans les exemples suivants.

Dans ces exemples, on utilise comme agent de contrôle de la polymérisation le nitroxyde stable, nommé SG1, de formule :

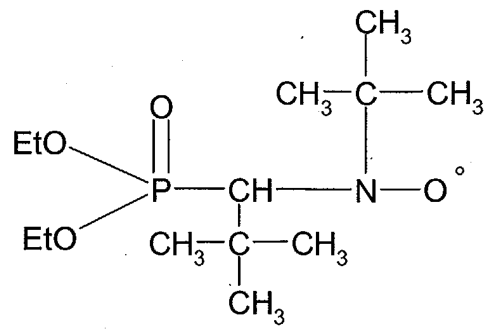

Les initiateurs de polymérisation mentionnés dans les exemples sont des alcoxyamines nommés 'DIAMS' et 'MONAMS' qui correspondent aux formules suivantes :

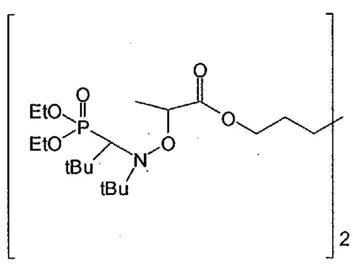

DIAMS

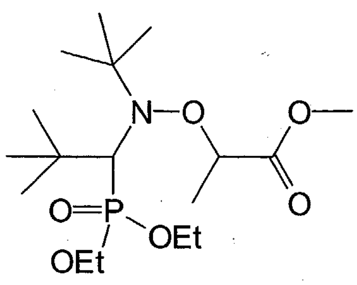

MONAMS

<u>**Exemple 1 : synthèse en masse de copolymère à gradient**</u>

Le mélange de réactifs est le suivant :

- MONAMS : 3,0 g
- SG1 : 0,18 g
- Acrylate d'éthyle : 480 g soit 80% en poids / poids total de monomères
- Styrène : 60 g soit 10% en poids / poids total de monomères
- Acide méthacrylique : 60 g soit 10% en poids / poids total de monomères

L'ensemble des constituants est mélangé, en l'absence de solvant, sous atmosphère d'azote, puis chauffé à une température maintenue entre 110 et 115°C pendant 198 minutes. La réaction est arrêtée à un taux de conversion

de 60%.

Le calcul du gradient par simulation donne la courbe ci-dessous. La prédiction théorique donne 30% d'incorporation du mélange (styrène/acide méthacrylique) et 70% d'acrylate d'éthyle.

La validité de ce modèle est donné par le suivi des concentrations relatives des trois monomères par chromatographie gazeuse et analyse RMN des polymères.

Par ces méthodes, on constate qu'à 60% de conversion, la composition chimique finale du copolymère est la suivante (% en poids): 68,4% d'acrylate d'éthyle, 16,1% de styrène et 15,5% d'acide méthacrylique selon la RMN sur la courbe calculée (69%).

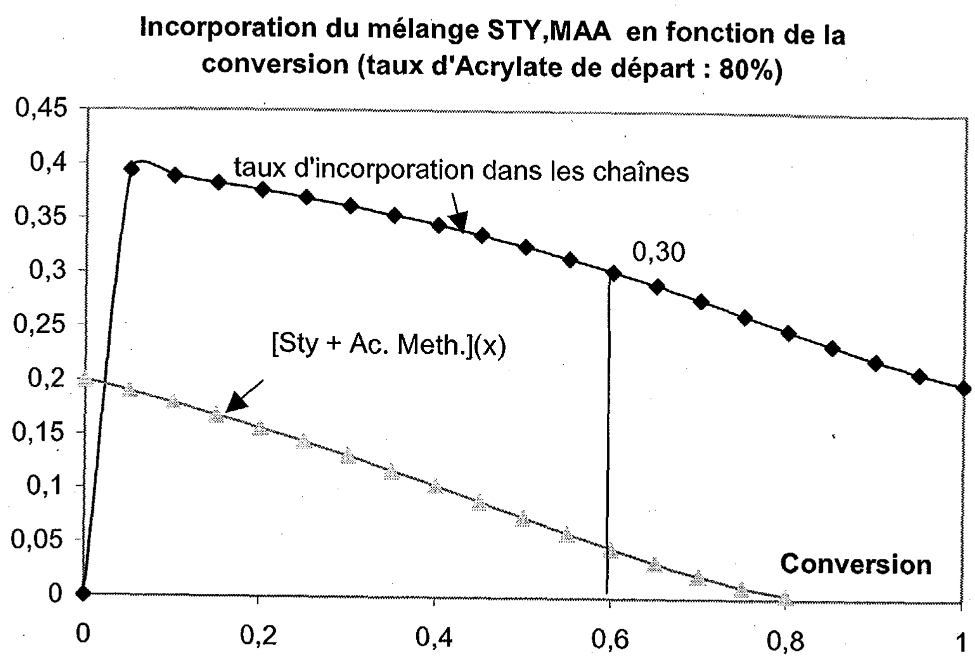


Par LAC, la trace du polymère montre la faible polydispersité de la composition chimique des chaînes.

La mesure des masses par chromatographie d'exclusion stérique conduit aux résultats suivants :

Mn= 32140 g/mol et Mw=51700 g/mol, d'où un indice de polydispersité Ip =1,6.

La dispersité en composition (ou w) est de 1,6.

Une représentation schématique du copolymère obtenu peut être la suivante :

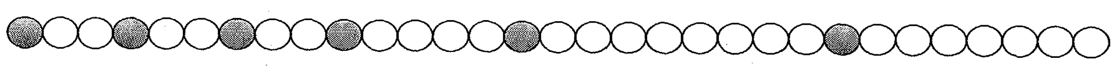

dans laquelle les sphères foncées désignent les enchaînements styrène/acide méthacrylique, et les sphères blanches désignent les enchaînements acrylate d'éthyle.

<u>**Exemple 2 : synthèse en masse de copolymère à gradient**</u>

Selon le mode opératoire décrit à l'exemple 1, on a préparé différents copolymères à partir du mélange de réactifs suivant :

- MONAMS : 3,0 g
- SG1:0,18g
- Styrène : 60 g
- Acide méthacrylique : 60 g
- Acrylate (ou mélange d'acrylate) : 480 g

| Exemple | Acrylate | Caractéristiques du copolymère | Composition finale du copolymère (% en poids) |
|---|---|---|---|
| 2a | Acrylate de butyle | Mn= 31100 g/mol<br>Mw= 52930 g/mol<br>Ip = 1,7 | Styrène :18<br>Ac. méthacrylique : 22<br>Acrylate de butyle : 60 |
| 2b | Acrylate de méthyle | Mn= 32750 g/mol<br>Mw= 61470 g/mol<br>Ip = 1,88 | Styrène :20<br>Ac. méthacrylique : 21<br>Acrylate de méthyle: 59 |
| 2c | Mélange 50/50 en poids acrylate de butyle/acrylate d'éthyle | Mn= 29690 g/mol<br>Mw= 51630 g/mol<br>Ip = 1,74 | Styrène :18<br>Ac. méthacrylique : 16<br>Acrylates : 66 |

**Exemple 3 : synthèse en présence de solvant**

La même synthèse qu'à l'exemple 1 est réalisée, mais en présence de solvant.
Le mélange de réactifs est le suivant :

- MONAMS : 3,43 g
- SG1 : 0,2 g
- Acrylate d'éthyle : 336 g
- Styrène : 42 g
- Acide méthacrylique : 42 g
- Toluène : 180 g

L'ensemble des constituants est mélangé, dans le toluène comme solvant, sous atmosphère d'azote, puis chauffé à une température maintenue entre 110 et 115°C, pendant 198 minutes.
Le taux de conversion final est de 82%, et le taux de solide obtenu est de 57,2% en poids.
On détermine les résultats analytiques ci-après :
Mn = 30570 g/mol, Mw= 50500 g/mol et Ip = 1,65.
La dispersité en composition (ou w) est de 2,0.
La composition finale du copolymère est donnée par chromatographie d'adsorption liquide (LAC), qui indique la similitude de composition avec le copolymère préparé à l'exemple 1 et l'absence d'homopolymère dans les matériaux.

**Exemple 4 : synthèse en présence de solvant**

On réalise, selon le procédé de l'exemple 3, à 120°C et durant 400 minutes, la synthèse d'un nouveau copolymère mais dans un solvant différent : la méthyléthylcétone.
La composition initiale du mélange est :

- MONAMS : 4,893 g
- SG1 : 0,2881 g
- Acrylate d'éthyle : 293,8 g
- Acrylate de méthyle : 32, 66 g
- Styrène : 76,8 g
- Acide méthacrylique : 76,8 g
- Méthyléthylcétone : 120 g

Le taux de conversion finale est de 99%, le taux de solide obtenu est de 79,9%.
On détermine les résultats analytiques ci-après :

Mn = 30500 g/mol
Mw= 58000 g/mol
Ip = 1,9

L'incorporation des monomères au cours du temps est mesurée en suivant par chromatographie gazeuse les taux de monomères résiduels (en %) au cours du temps (en minute):

| | | | | | | | |
|---|---|---|---|---|---|---|---|
| temps | | 0 | 75 | 130 | 190 | 290 | 400 |
| Conversion Globale | | 0 | 16 | 30,5 | 49,5 | 85,4 | 99 |
| residuels (%) | AMe | 5,45 | 5,1 | 3,75 | 3,75 | 1,6 | 0,13 |
| | AE | 48,95 | | | | 17,95 | 1,2 |
| | AMA | 12,8 | 12,15 | 4,6 | 2 | 0,35 | 0,08 |
| | S | 12,8 | 12,46 | 6,7 | 3,92 | 0,15 | 0,007 |

- Acrylate d'éthyle : AE
- Acrylate de méthyle : AMe
- Styrène : S
- Acide méthacrylique AMA

Le taux de résiduel total est calculé en tenant compte du solvant quantifié par le taux de solide.

On note que chaque monomère est présent tout au long de la réaction. Le gradient déterminé pour chaque monomère peut alors être calculé et donne les courbes suivantes :

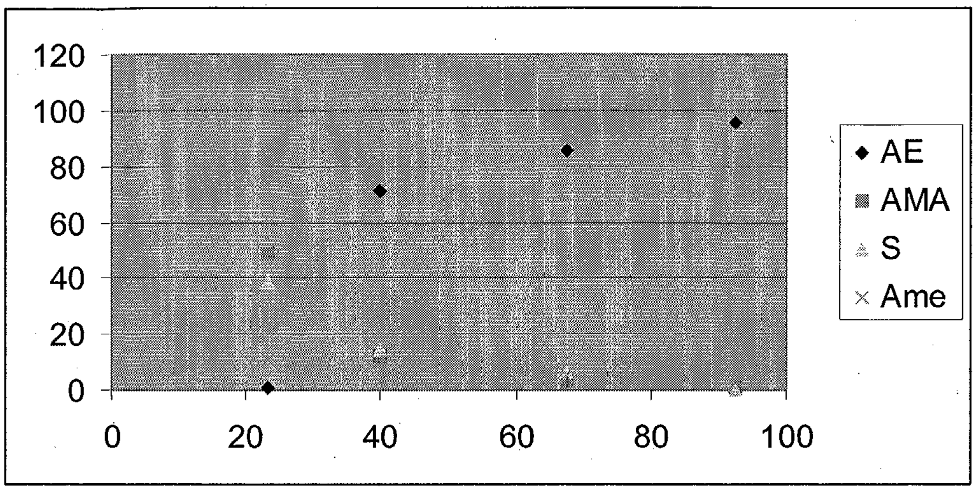


La composition finale du copolymère est la suivante :

- acrylate d'éthyle : 34% en poids
- acrylate de méthyle : 34% en poids
- styrène : 16% en poids
- acide méthacrylique : 16% en poids

**Exemple 5**

On prépare une dispersion aqueuse à 10% de taux de solide, du copolymère préparé à l'exemple 2a. Pour ce faire, le polymère est préalablement séché à l'étuve. Puis, on dissout 10 g de polymère dans 90 ml d'eau comprenant 1,6 g d'AMP (amino-2-méthyl-2-propanol). On obtient une dispersion aqueuse limpide et très fluide. La taille des particules, mesurée par diffusion (appareil Coulter 4NW) est de 33 nm.

**Exemple 6**

On prépare une dispersion aqueuse du copolymère préparé à l'exemple 1.
On dissout 10 g de polymère dans 40 g de tétrahydrofurane; on ajoute 1,41 g d'AMP (amino-2-méthyl-2- propanol) dissous dans 10 ml d'eau. La solution s'épaissit. On ajoute alors lentement et sous vive agitation 90 ml d'eau déminéralisée. La solution reste limpide et redevient fluide.
Le solvant est évaporé et on obtient une dispersion aqueuse limpide et fluide. La taille des particules, mesurée par diffusion (appareil Coulter 4NW) est de 199 nm.

## Exemple 7

On prépare des dispersions aqueuses à 4,6% en poids de matière sèche à partir des dispersions des exemples 5 et 6.
On prépare ensuite un aérosol comprenant :

- 65 g de dispersion aqueuse de polymère à 4,6% en poids de matière sèche
- 35 g de DME (diméthyléther)

La laque présente une bonne sprayabilité, ainsi qu'un bon pouvoir laquant.

## Exemple 8

On détermine le module de Young, la déformation à la rupture et la recouvrance élastique instantanée pour les copolymères des exemples 5 et 6.
On obtient après séchage un film qui a les caractéristiques suivantes, mesurées selon les protocoles décrits précédemment :

| | Module de Young | Déformation à la rupture $\varepsilon_r$ | Recouvrance élastique instantanée $\varepsilon_i$ |
|---|---|---|---|
| Exemple 5 | 0,5 ± 0,1 MPa | 1000 ± 200% | 40 ± 1% |
| Exemple 6 | 2 ± 0,1 MPa | 480 ± 50% | 39 ± 2% |

## Revendications

1. Composition cosmétique capillaire comprenant, dans un milieu cosmétiquement acceptable, au moins un copolymère filmogène à gradient comprenant au moins deux monomères différents, et présentant un indice de polydispersité en masse (Ip) inférieur ou égal à 2,5 de préférence compris entre 1,1 et 2,3, notamment entre 1,15 et 2,0, voire entre 1,2 et 1,9 ou 1,8, ladite composition étant apte à former un film présentant au moins une des caractéristiques suivantes:

   - une déformation à la rupture $\varepsilon_r$ comprise entre 5% et 2500%, de préférence entre 10% et 2000% et encore mieux entre 15% et 1000%, et/ou
   - un module d'Young compris entre 0,5 et 1200 MPa, de préférence entre 1 et 1000 MPa, préférentiellement entre 2 et 800 MPa, et/ou
   - une recouvrance élastique instantanée $\varepsilon_i$ supérieure ou égale à 10%, de préférence supérieure ou égale à 25%, et mieux supérieure ou égale à 35%, notamment comprise entre 10-100%, par exemple 25-98%, voire 35-95%.

2. Composition selon la revendication 1, dans laquelle la masse moléculaire moyenne en poids du copolymère à gradient est de préférence comprise entre 5000 g/mol et 1 000 000 g/mol, notamment entre 5500 g/mol et 800 000 g/mol, et encore mieux entre 6000 g/mol et 500 000 g/mol.

3. Composition selon l'une des revendications précédentes, dans laquelle la masse moléculaire moyenne en nombre du copolymère à gradient est comprise entre 5000 g/mol et 1 000 000 g/mol, notamment entre 5500 g/mol et 800 000 g/mol, et encore mieux entre 6000 g/mol et 500 000 g/mol.

4. Composition selon l'une des revendications précédentes, dans laquelle le copolymère est tel que toutes les chaînes polymériques ont au moins un monomère Mi pour lequel, quelque soit la position normalisée x sur la chaîne polymérique, il y a une probabilité non nulle de retrouver ce monomère Mi le long de la chaîne.

5. Composition selon l'une des revendications précédentes, dans laquelle le copolymère est tel que sur la courbe de chromatographie d'adsorption (LAC), représentant la proportion de polymères en fonction du volume d'élution,

la différence ($V^{1/2}$ max - $V^{1/2}$ min) est inférieure ou égale à 3,5, notamment comprise entre 1 et 2,8 et mieux entre 1,2 et 2,5, avec "$V^{1/2}$ min" étant la valeur minimum du volume d'élution à mi-hauteur de la courbe, et "$V^{1/2}$ max" étant la valeur maximum du volume d'élution à mi-hauteur de la courbe.

**6.** Composition selon l'une des revendications précédentes, dans laquelle le copolymère à gradient comprend au moins deux monomères différents, qui sont présents chacun à raison de 1 à 99% en poids par rapport au copolymère final, notamment à raison de 2-98% en poids, de préférence à raison de 5-95% en poids.

**7.** Composition selon l'une des revendications précédentes, dans laquelle le copolymère à gradient comprend au moins un monomère hydrophile, ou un mélange de monomères hydrophiles, qui peut être présent à raison de 1 à 99% en poids, notamment 2-70% en poids, encore mieux 5-50% en poids, voire 10-30% en poids, par rapport au poids total du copolymère.

**8.** Composition selon l'une des revendications précédentes, dans laquelle le copolymère à gradient comprend au moins un monomère dont l'homopolymère a une Tg inférieure ou égale à 20°C, notamment comprise entre -150°C et 20°C, plus particulièrement comprise entre -130°C et 18°C, et encore mieux comprise entre -120°C et 15°C, ou un mélange de tels monomères.

**9.** Composition selon la revendication 8, dans laquelle le monomère dont l'homopolymère a une Tg $\leq$ 20°C est présent à raison de 1 à 99% en poids, notamment 10-90% en poids, encore mieux 20-80% en poids, voire 50-75% en poids, par rapport au poids total du copolymère.

**10.** Composition selon l'une des revendications précédentes, dans laquelle le copolymère à gradient comprend au moins un monomère hydrophile choisi parmi :

- les dérivés de (méth)acrylates d'aminoalkyles en C1-C6, et notamment les (méth)acrylates N,N-dialkyle en C1-C4 aminoalkyle en C1-C6 tels que le méthacrylate de N,N-diméthylaminoéthyle (MADAME) ou le méthacrylate de N,N-diéthylaminoéthyle (DEAMEA) ;
- les (méth)acrylamides N,N-dialkyle en C1-C4 éventuellement aminoalkyle en C1-C6, tels que la N,N-diméthylacrylamide, le N,N-diméthylaminopropylacrylamide (DMAPA) ou le N,N-diméthylaminopropylmethacrylamide (DMAPMA),
- les dialkyl dallylamines en C1-C8 tels que la diméthyldiallylamine;
- la vinylamine;
- les vinylpyridines et notamment la 2-vinylpyridine ou la 4-vinylpiridine ;
  ainsi que leurs sels d'acides minéraux, ou d'acide organique, ou leurs formes quaternisées.
- les acides carboxyliques, notamment mono- ou di-carboxyliques, éthyléniques tels que l'acide acrylique, l'acide méthacrylique, l'acide crotonique, l'acide itaconique, l'acide fumarique, l'acide maléique ;
- les anhydrides carboxyliques porteurs d'une liaison vinylique tels que l'anhydride maléique
- les acides sulfoniques éthyléniques tels que l'acide styrènesulfonique, l'acide acrylamidopropanesulfonique, et leurs sels,
- l'acide vinylbenzoïque, l'acide vinylphosphonique et leurs sels;
- le sel de potassium de l'acryloyloxy-3-sulfopropyle, ou le composé de formule $CH_2=CHCOOCH_2OCH_2(OH)CH_2SO_3^-Na^+$
- les amides des acides carboxyliques insaturés comme l'acrylamide ou le méthacrylamide et leur dérivés N-substitués tels que les (méth)acrylamides de N-alkyle en $C_1$-$C_4$ comme la N-méthylacrylamide; les (méth) acrylamides de N,N-dialkyle $C_1$-$C_4$, comme la N,N-diméthylacrylamide;
- les (méth)acrylates d'hydroxyalkyle notamment ceux dont le groupe alkyle a de 2 à 4 atomes de carbone, en particulier le (méth)acrylate d'hydroxyéthyle;
- les (méth)acrylates de polyéthylène glycol (5 à 100 OE) ou de glycol substitués ou non sur leur fonction terminale par des groupements alkyl, phosphates, phosphonates, sulfonate, par exemple l'acrylate de glycérol, le (méth)acrylate de methoxypolyethylene glycol (8 ou 12OE); le (méth)acrylate d'hydroxypolyethylene glycol ;
- les (méth)acrylates d'alkoxyalkyle tels que le (méth)acrylate d'éthoxyéthyle;
- les (méth)acrylates de polysaccharide comme l'acrylate de saccharose.
- les vinylamides telles que la vinyl acétamide; éventuellement cycliques en particulier les vinyllactames tels que la N-vinylpyrrolidone ou la N-vinylcaprolactame;
- les vinyléthers tels que le vinylmethyléther.
- le methacrylamidopropoxy trimethylammoniumbetaine;
- le N,N-dimethyl-N-methacryloxyéthyl-N-(3-sulfopropyl)ammoniumbetaine,

- le 3-methacryloylethoxycarbonylpyridinium
- le composé de formule :

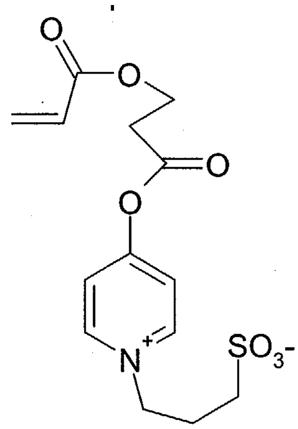

- le 4-vinylpyridyniumsulfopropylbetaine de formule :

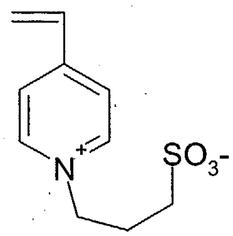

**11.** Composition selon l'une des revendications précédentes, dans laquelle le copolymère à gradient comprend au moins un monomère hydrophile choisi parmi le méthacrylate de N,N-diméthylaminoéthyle (MADAME), l'acide acrylique, l'acide méthacrylique, l'acide crotonique, l'acide styrènesulfonique, l'acide acrylamidopropanesulfonique, le diméthylaminopropylmethacrylamide (DMAPMA); le styrène sulfonate, l'acrylate d'hydroxyéthyle, l'acrylate de glycérol, le méthacrylate d'éthoxyéthyle, l'acrylate d'éthoxyéthyle, le (méth)acrylate de methoxypolyethylene glycol (8 ou 12OE); le (méth)acrylate d'hydroxypolyethylene glycol ; la N-vinylpyrrolidone, la N-vinylcaprolactame, l'acrylamide, la N,N-dimethylacrylamide.

**12.** Composition selon l'une des revendications précédentes, dans laquelle le copolymère à gradient comprend au moins un monomère choisi parmi les (méth)acrylates d'alkyles en C1-C4, tels que le (méth)acrylate de tertio-butyle ou le (méth)acrylate d'éthyle, qui conduisent à l'obtention de l'acide (méth)acrylique après hydrolyse.

**13.** Composition selon l'une des revendications précédentes, dans laquelle le copolymère à gradient comprend au moins un monomère dont l'homopolymère a une Tg inférieure ou égale à 20°C, choisi parmi :

- les hydrocarbures éthyléniques de 2 à 10 carbone, tels que l'éthylène, l'isoprène, et le butadiène ;
- les acrylates de formule $CH_2 = CHCOOR_1$, $R_1$ représentant un groupe hydrocarboné saturé ou non, de 1 à 12 carbones, linéaire ou ramifié à l'exception du groupe tertio-butyle, dans lequel se trouve(nt) éventuellement intercalé(s) un ou plusieurs hétéroatomes choisis parmi O, N, S, Si, ledit groupe alkyle pouvant en outre être éventuellement substitué par un ou plusieurs substituants choisis parmi les groupes hydroxyle et les atomes d'halogène (Cl, Br, I et F) ;
  $R_1$ peut aussi être un groupement $-(R'')x-(OC_2H_4)_n-OR'''$, avec x = 0 ou 1, R" = groupe hydrocarboné saturé ou non, de 1 à 12 carbones, linéaire ou ramifié, n = 5 à 100 et R''' = H ou $CH_3$;
- les méthacrylates de formule : $CH_2=C(CH_3)-COOR_2$ , dans laquelle $R_2$ représente un groupe hydrocarboné saturé ou non, de 3 à 12 carbones, linéaire ou ramifié, dans lequel se trouve(nt) éventuellement intercalé(s) un ou plusieurs hétéroatomes choisis parmi O, N, S et Si, ledit groupe alkyle pouvant en outre être éventuellement substitué par un ou plusieurs substituants choisis parmi les groupes hydroxyle et les atomes d'halogènes (Cl, Br, I, F) ; $R_2$ peut aussi être un groupement $-(R'')x-(OC_2H_4)_n-OR'''$, avec x = 0 ou 1, R" = groupe

hydrocarboné saturé ou non, de 1 à 12 carbones, linéaire ou ramifié, n = 5 à 100 et R''' = H ou $CH_3$;

- les dérivés N ou N,N-substitués des amides d'acides carboxyliques insaturés en C1-12, notamment les (méth) acrylamides de N-alkyle en C1-12, tel que le N-octylacrylamide;
- les esters de vinyle de formule : $R_3$-CO-O-CH = $CH_2$ où $R_3$ représente un groupe alkyle de 2 à 12 carbone, linéaire ou ramifié, notamment le propionate de vinyle, le butyrate de vinyle, l'éthylhexanoate de vinyle, le néononanoate de vinyle, et le néododécanoate de vinyle ;
- les éthers de vinyle et d'alkyle ayant 1 à 12 carbones tels que l'éther de vinyle et de méthyle, et l'éther de vinyle et d'éthyle ;

**14.** Composition selon l'une des revendications précédentes, dans laquelle le copolymère à gradient comprend au moins un monomère dont l'homopolymère a une Tg inférieure ou égale à 20°C, choisi parmi :

- l'isoprène et le butadiène ;
- les acrylates de méthyle, d'éthyle, d'isobutyle, de n-butyle, d'éthylhexyle; de méthoxyéthyle, d'éthoxyéthyle; d'hydroxypolyéthylèneglycol;
- les méthacrylates d'éthoxyéthyle, d'hexyle, d'éthylhexyle; d'hydroxypolyéthylèneglycol;
- les (méth)acrylamides de N-alkyle en C6-12, tel que le N-octylacrylamide;
- les esters de vinyle de formule : $R_3$-CO-O-CH = $CH_2$ où $R_3$ représente un groupe alkyle de 6 à 12 carbones, linéaire ou ramifié, notamment le néononanoate de vinyle et le néododécanoate de vinyle.

**15.** Composition selon l'une des revendications précédentes, dans laquelle le copolymère à gradient comprend au moins un monomère dont l'homopolymère a une Tg supérieure ou égale à 20°C, choisi parmi :

- les composés vinyliques de formule : $CH_2$ = CH-$R_4$, où $R_4$ est un groupe hydroxyle ; un groupe -NH-C(O) -$CH_3$, un groupe -OC(O)-$CH_3$, un groupe cycloalkyle en $C_3$ à $C_8$ ; un groupe aryle en $C_6$ à $C_{20}$ ; un groupe aralkyle en $C_7$ à $C_{30}$ (groupe alkyle en $C_1$ à $C_4$) ; un groupe hétérocyclique de 4 à 12 chaînons contenant un ou plusieurs héréatomes choisis parmi O, N, et S ; un groupe hétérocyclylalkyle (alkyle en $C_1$ à $C_4$) tel qu'un groupe furfuryle ; lesdits groupes cycloalkyle, aryle, aralkyle, hétérocyclique, ou hétérocyclylalkyle pouvant être éventuellement substitué par un ou plusieurs substituants choisis parmi les groupes hydroxyles, les atomes d'halogène, et les groupes alkyles de 1 à 4 carbone, linéaires ou ramifiés dans lesquels se trouve(nt) éventuellement intercalé(s) un ou plusieurs hétéroatomes choisis parmi O, N, S et P, et lesdits groupes alkyle pouvant, en outre, être éventuellement substitués par un ou plusieurs substituants choisis parmi les groupes hydroxyles, et les atomes d'halogène (Cl, Br, I et F) ou Si.
- les acrylates de formule $CH_2$ = CH-$COOR_5$, où $R_5$ est un groupe tertiobutyle, un groupe cycloalkyle en $C_3$ à $C_8$ ; un groupe aryle en $C_6$ à $C_{20}$ ; un groupe aralkyle en $C_7$ à $C_{30}$ (groupe alkyle en $C_1$ à $C_4$) ; un groupe hétérocyclique de 4 à 12 chaînons contenant un ou plusieurs hétéroatomes choisis parmi O, N, et S ; un groupe hétérocyclylalkyle (alkyl de $C_1$ à $C_4$), tel qu'un groupe furfuryle ; lesdits groupes cycloalkyle, aryle, aralkyle, hétérocyclique ou hétérocyclylalkyle pouvant être éventuellement substitué par un ou plusieurs substituants choisis parmi les groupes hydroxyles, les atomes d'halogène, et les groupes alkyles de 1 à 4 C linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle et les atomes d'halogène (Cl, Br, I et F) ou Si.
- les méthacrylates de formule $CH_2$ = C($CH_3$)-$COOR_6$, où $R_6$ est un groupe alkyle de 1 à 4C, linéaire ou ramifié, tel qu'un groupe méthyle, éthyle, propyle ou isobutyle, ledit groupe alkyle pouvant en outre être éventuellement substitué par un ou plusieurs substituants choisis parmi les groupes hydroxyle et les atomes d'halogène (Cl, Br, I et F) ou Si; un groupe cycloalkyle en $C_3$ à $C_8$ ; un groupe aryle en $C_6$ à $C_{20}$ ; un groupe aralkyle en $C_7$ à $C_{30}$ (groupe alkyle en $C_1$ à $C_4$) ; un groupe hétérocyclique de 4 à 12 chaînons contenant un ou plusieurs hétéroatomes choisis parmi O, N, et S ; un groupe hétérocyclylalkyle (alkyle de 1 à 4 C), tel qu'un groupe furfuryle ; lesdits groupes cycloalkyle, aryle, aralkyle, ou hétérocyclique ou hétérocyclylalkyle pouvant être éventuellement substitué par un ou plusieurs substituants choisis parmi les groupes hydroxyles, les atomes d'halogène, et les groupes alkyles de 1 à 4 carbone, linéaires ou ramifiés, dans lequel se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle et les atomes d'halogène (Cl, Br, I et F).
- les (méth)acrylamides de formule : $CH_2$=C(R')-CO-$NR_7R_8$,
  où $R_7$ et $R_8$ identiques ou différents représentent un atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle, et R' désigne H ou méthyle.

**16.** Composition selon l'une des revendications précédentes, dans laquelle le copolymère à gradient comprend au moins un monomère dont l'homopolymère a une Tg supérieure ou égale à 20°C, choisi parmi :

- les acrylates de furfuryle, d'isobornyle, de tertiobutyle, de tertiobutylecyclohexyle, de tertiobutylbenzyle;
- les méthacrylates de méthyle, de n-butyle, d'éthyle, d'isobutyle,
- le styrène, le styrène sulfonate;
- l'acétate de vinyle et le vinylcyclohexane.

**17.** Composition selon l'une des revendications précédentes, dans laquelle le copolymère à gradient est présent en une quantité de 0,1 à 60% en poids, de préférence 0,5 à 40% en poids, notamment 1 à 35% en poids, voire 5-30% en poids, par rapport au poids total de la composition.

**18.** Composition selon l'une des revendications précédentes, dans laquelle le copolymère à gradient est présent sous forme solubilisée, par exemple dans l'eau ou un solvant organique, ou bien sous forme de dispersion aqueuse ou organique.

**19.** Composition selon l'une des revendications précédentes, comprenant au moins un constituant choisi parmi l'eau; les solvants organiques; les polymères filmogènes anioniques, cationiques, non-ioniques ou amphotères; les polymères fixants; les silicones volatiles ou non volatiles; les agents tensioactifs anionique, cationiques, amphotères ou non-ioniques, les agents épaississants, les agents nacrants, les filtres UV, les agents anti-radicalaires, les parfums, les agents conservateurs, les pigments et colorants, les agents d'ajustement du pH, les agents solubilisants, les agents plastifiants, les agents anti-mousse, les cires et huiles, les vitamines, des agents conditionneurs et les particules organiques ou minérales, synthétiques ou d'origine naturelle.

**20.** Composition aérosol conditionnée dans un dispositif aérosol, comprenant au moins un agent propulseur et au moins une composition capillaire telle que définie dans l'une des revendications 1 à 19.

**21.** Composition selon la revendication 20, dans laquelle l'agent propulseur est choisi parmi le diméthyléther; les alcanes en $C_{3-5}$, tels que le propane, le n-butane et l'isobutane; le 1,1-difluoroéthane, les mélanges de diméthyléther et d'alcanes en $C_{3-5}$, et les mélanges de 1,1-difluoroéthane et de diméthyléther et/ou d'alcanes en $C_{3-5}$.

**22.** Composition selon l'une des revendications précédentes, se présentant sous la forme d'un produit capillaire pour le maintien de la coiffure ou la mise en forme des cheveux, notamment sous la forme de shampooings, de gels, de lotions de mise en plis, de lotions pour le brushing, de compositions de fixation et de coiffage telles que les laques ou spray.

**23.** Procédé de traitement, notamment de coiffage, des cheveux, consistant à appliquer sur ceux-ci la composition capillaire telle que définie à l'une des revendications 1 à 19, ou à vaporiser sur ceux-ci la composition aérosol telle que définie à l'une des revendications 20 à 21, et éventuellement à laisser sécher la chevelure ainsi traitée.